# EUROPEAN PATENT APPLICATION

(11) **EP 4 772 229 A2**
(43) Date of publication of application: **08.07.2026**
(21) Application number: 26169175.2
(22) Date of filing: 23.12.2024
(51) Int. Cl.: A61M 25/00

(54) **INLET GUARDS FOR BLOOD PUMPS**

(30) Priority: 28.12.2023 US 202363615377 P; 18.03.2024 US 202463566681 P; 10.09.2024 US 202463692734 P
(62) Divisional of application: 24841310.6
(71) Applicant: Magenta Medical Ltd., 6092000 Kadima (IL)
(72) Inventor: TUVAL, Yosi, 4050426 Even Yehuda (IL); LUBINSKY, Gad, 4069600 Ein Vered (IL); SUDIN, Yuri, 7176053 Modi'in-Makkabbim-Re'ut (IL); ROZENFELD, Avi, 3460205 Haifa (IL); MUSTACCHI, Shaul, 4805638 Rosh Haayin (IL); TAMIR, Ran, 6248429 Ramat Gan (IL); ZIPORY, Yuval, 7175472 Modiin (IL)
(74) Representative: Evens, Paul Jonathan

(57) **Abstract**

An apparatus, comprising: a blood pump (20), comprising: a frame (34) comprising a proximal portion (36), a central portion (38) comprising multiple struts (37), and a distal portion (40); an impeller (50), which is configured to pump blood of a subject proximally, disposed within the frame (34); a pump-outlet tube (24), which is fixed over the proximal portion (36) of the frame (34) and at least part of the central portion (38) of the frame (34); and an inlet guard (145), which: is distal to the impeller (50), comprises a main body shaped to define one or more blood-inlet openings (108) configured to allow passage of the blood therethrough, and comprises multiple proximal flaps (146), which are coupled to the pump-outlet tube (24) and are fixed at least partly over the central portion (38) of the frame (34) without overlapping any of the struts (37).

## Description

### CROSS-REFERENCE TO RELATED APPLICATIONS

The present application claims priority from US Provisional Application 63/615,377 to Tuval et al., entitled "Inlet guards for blood pumps," filed December 28, 2023, which is incorporated herein by reference, US Provisional Application 63/566,681 to Tuval et al., entitled "Inlet guards for blood pumps," filed March 18, 2024, which is incorporated herein by reference, and US Provisional Application 63/692,734 to Tuval et al., entitled "Inlet guards for blood pumps," filed September 10, 2024, which is incorporated herein by reference.

### FIELD OF EMBODIMENTS

Some embodiments relate generally to medical devices, and specifically to blood pumps, e.g., for ventricular assist devices.

### BACKGROUND

Ventricular assist devices are mechanical circulatory support devices designed to assist and unload cardiac chambers in order to maintain or augment cardiac output. They are used in patients suffering from a failing heart and in patients at risk for deterioration of cardiac function during percutaneous coronary interventions. Most commonly, a left-ventricular assist device is applied to a defective heart in order to assist left-ventricular functioning. In some cases, a right-ventricular assist device is used in order to assist right-ventricular functioning. Such ventricular assist devices are either designed to be permanently implanted or mounted on a catheter for temporary placement.

### SUMMARY

Some embodiments of the present disclosure include a blood pump including an inlet guard. The inlet guard includes a main body, which is typically frustoconical and is shaped to define one or more blood-inlet openings configured to allow passage of blood therethrough. The inlet guard further includes multiple proximal flaps extending proximally from the main body. The blood pump further includes a frame including a proximal portion, a central portion, and a distal portion, an inner lining that lines at least part of the central portion of the frame, an impeller, which is configured to pump blood proximally, disposed within the frame, and a pump-outlet tube, which is fixed over the proximal portion of the frame and at least part of the central portion of the frame, and which is heat welded to the inner lining. The inlet guard is distal to the impeller, with the proximal flaps of the inlet guard being disposed between the pump-outlet tube and the inner lining where the pump-outlet tube and the inner lining are heat welded to one another. To facilitate coupling the inlet guard in this manner, the inlet guard is typically made of a material having a glass-transition temperature that is higher than respective glass-transition temperatures of each of the inner lining and the pump-outlet tube. Typically, the frame defines struts and the proximal flaps do not overlap any of the struts of the frame.

In some embodiments, to manufacture a frustoconical inlet guard, the blood-inlet openings are formed in a sheet of material, and the sheet of material is then rolled into the frustoconical shape. The inlet guard is coupled to the pump-outlet tube, e.g., via proximal flaps as described above, and is fixed over the distal portion of the frame.

Other embodiments include an inflatable element, various embodiments of which are described herein. The device further includes a pump-outlet tube, which in some embodiments includes a lateral wall shaped to define one or more blood-outlet openings and is configured for insertion, through an aorta of a subject, into a left ventricle of a heart of the subject such that the blood-outlet openings are disposed within the aorta and a distal portion of the pump-outlet tube is disposed within the left ventricle. The device further includes an impeller configured to pump blood of the subject proximally through the pump-outlet tube, such that the blood exits the pump-outlet tube via the blood-outlet openings. The device further includes a delivery tube configured to extend, from outside the body of the subject, through the pump-outlet tube to the distal portion, and a drive cable passing through the delivery tube and configured to rotate the impeller. The inflatable element surrounds the delivery tube proximally to the blood-outlet openings. Advantageously, the inflatable element helps prevent injury to the aortic wall and/or centers the proximal portion of the pump-outlet tube in the aorta.

There is therefore provided, in accordance with some embodiments, a method for manufacturing a blood pump. The method includes inserting an impeller, which is configured to pump blood of a subject, into a frame including a proximal portion, a central portion, and a frustoconical distal portion. The method further includes fixing a pump-outlet tube over the proximal portion of the frame and at least part of the central portion of the frame. The method further includes forming one or more blood-inlet openings in a sheet of material, and subsequently to forming the blood-inlet openings, rolling the sheet of material so as to form a frustoconical inlet guard. The method further includes coupling the inlet guard to the pump-outlet tube, and fixing the inlet guard over the distal portion of the frame.

In some embodiments,
one or more tabs extend from the sheet of material,
rolling the sheet of material includes rolling the sheet of material by pulling the tabs, and
the method further includes, subsequently to rolling the sheet of material, removing the tabs from the sheet of material.

In some embodiments, the pump-outlet tube is configured to traverse an aortic valve of the subject, and the impeller is configured to pump the blood from a left ventricle of a heart of the subject, through the pump-outlet tube, into an aorta of the subject.

In some embodiments, the blood-inlet openings are sized such that the inlet guard is configured to block chordae tendineae, trabeculae carneae, and papillary muscles of a left ventricle of a heart of the subject from entering the frame.

In some embodiments, for each of the blood-inlet openings, a span of the blood-inlet opening in at least one direction is less than 1 mm.

In some embodiments, an area of each of the blood-inlet openings is 0.05-5 mm².

In some embodiments, forming the blood-inlet openings includes forming the blood-inlet openings such that a porosity of the inlet guard is at least 40 percent.

In some embodiments, each of the blood-inlet openings is hexagonal.

In some embodiments, forming the blood-inlet openings includes forming the blood-inlet openings such that a distance between each pair of adjacent ones of the blood-inlet openings is 0.01-0.1 mm.

In some embodiments, fixing the inlet guard over the distal portion of the frame includes fixing the inlet guard over the distal portion of the frame by coupling the inlet guard to the distal portion of the frame.

In some embodiments, the method further includes forming multiple proximal flaps in the sheet of material, and coupling the inlet guard to the pump-outlet tube includes coupling the inlet guard to the pump-outlet tube by coupling the proximal flaps to the pump-outlet tube.

In some embodiments, fixing the inlet guard over the distal portion of the frame includes fixing the inlet guard over the distal portion of the frame by fixing the proximal flaps at least partly over the central portion of the frame.

In some embodiments, the central portion of the frame includes multiple struts, and forming the proximal flaps includes shaping the proximal flaps such that, when the proximal flaps are fixed at least partly over the central portion of the frame, the proximal flaps do not overlap any of the struts.

In some embodiments, shaping the proximal flaps includes shaping the proximal straps such that, when the proximal flaps are fixed at least partly over the central portion of the frame, the proximal flaps fit between the struts while abutting the struts.

In some embodiments, the method further includes:
lining at least part of the central portion of the frame with an inner lining; and
coupling the inlet guard to the inner lining.

In some embodiments, the method further includes forming multiple proximal flaps in the sheet of material,
coupling the inlet guard to the pump-outlet tube includes coupling the inlet guard to the pump-outlet tube by coupling the proximal flaps to the pump-outlet tube, and
coupling the inlet guard to the inner lining includes coupling the inlet guard to the inner lining by coupling the proximal flaps to the inner lining, such that the proximal flaps are between the pump-outlet tube and the inner lining.

In some embodiments, coupling the proximal flaps to the pump-outlet tube and to the inner lining includes coupling the proximal flaps to the pump-outlet tube and to the inner lining by heat welding the pump-outlet tube to the inner lining while the proximal flaps are between the pump-outlet tube and the inner lining.

In some embodiments, the method further includes forming multiple flap openings in the proximal flaps, and heat welding the pump-outlet tube to the inner lining includes heat welding the pump-outlet tube to the inner lining at least partly via the flap openings.

In some embodiments, a glass-transition temperature of the material is higher than respective glass-transition temperatures of each of the inner lining and the pump-outlet tube, and heat welding the pump-outlet tube to the inner lining includes heat welding the pump-outlet tube to the inner lining at a heat-welding temperature that is lower than the glass-transition temperature of the material but higher than the glass-transition temperatures of each of the inner lining and the pump-outlet tube.

In some embodiments,
the inner lining is made of a polyurethane,
the pump-outlet tube is made of a polyether block amide, and
the material is a polyether ether ketone.

In some embodiments,
the inner lining and the pump-outlet tube are made of the same type or different types of polyurethane, and
the material is a polyether ether ketone.

In some embodiments,
inserting the impeller into the frame includes inserting the impeller into the frame while the impeller is disposed over an axial shaft configured to rotate,
the method further includes coupling a bearing housing, which houses a radial bearing configured to radially stabilize the axial shaft while the axial shaft rotates, to the frame distally to the frame, and
fixing the inlet guard over the distal portion of the frame includes fixing the inlet guard over the distal portion of the frame by coupling the inlet guard to the bearing housing.

In some embodiments, the method further includes forming multiple distal flaps in the sheet of material, and coupling the inlet guard to the bearing housing includes coupling the inlet guard to the bearing housing by coupling the distal flaps to the bearing housing.

There is further provided, in accordance with some embodiments, an apparatus including a blood pump. The blood pump includes a frame including a proximal portion, a central portion, and a frustoconical distal portion. The blood pump further includes an impeller, which is configured to pump blood of a subject, disposed within the frame. The blood pump further includes a pump-outlet tube, which is fixed over the proximal portion of the frame and at least part of the central portion of the frame. The blood pump further includes a frustoconical inlet guard, which includes a rolled sheet of material shaped to define one or more blood-inlet openings, which is coupled to the pump-outlet tube, and which is fixed over the distal portion of the frame.

There is further provided, in accordance with some embodiments, an apparatus including a blood pump. The blood pump includes a frame including a proximal portion, a central portion, and a distal portion. The blood pump further includes an inner lining that lines at least part of the central portion of the frame, an impeller, which is configured to pump blood of a subject proximally, disposed within the frame, and a pump-outlet tube, which is fixed over the proximal portion of the frame and at least part of the central portion of the frame, and which is heat welded to the inner lining. The blood pump further includes an inlet guard, which is distal to the impeller, includes a main body shaped to define one or more blood-inlet openings configured to allow passage of the blood therethrough, includes multiple proximal flaps extending proximally from the main body and disposed between the pump-outlet tube and the inner lining where the pump-outlet tube and the inner lining are heat welded to one another, and is made of a material having a glass-transition temperature that is higher than respective glass-transition temperatures of each of the inner lining and the pump-outlet tube.

In some embodiments, the proximal flaps are shaped to define multiple flap openings, and the pump-outlet tube and the inner lining are heat welded to one another at least partly via the flap openings.

In some embodiments,
the inner lining is made of a polyurethane,
the pump-outlet tube is made of a polyether block amide, and
the material is a polyether ether ketone.

In some embodiments,
the inner lining and the pump-outlet tube are made of the same type or different types of polyurethane, and
the material is a polyether ether ketone.

In some embodiments, the pump-outlet tube is configured to traverse an aortic valve of the subject, and the impeller is configured to pump the blood from a left ventricle of a heart of the subject, through the pump-outlet tube, into an aorta of the subject.

In some embodiments, the blood-inlet openings are sized such that the inlet guard is configured to block chordae tendineae, trabeculae carneae, and papillary muscles of a left ventricle of a heart of the subject from entering the frame.

In some embodiments, for each of the blood-inlet openings, a span of the blood-inlet opening in at least one direction is less than 1 mm.

In some embodiments, an area of each of the blood-inlet openings is 0.05-5 mm².

In some embodiments, a porosity of the inlet guard is at least 40 percent.

In some embodiments, each of the blood-inlet openings is hexagonal.

In some embodiments, a distance between each pair of adjacent ones of the blood-inlet openings is 0.01-0.1 mm.

In some embodiments, the inlet guard is coupled to the distal portion of the frame.

In some embodiments, the proximal flaps are fixed at least partly over the central portion of the frame.

In some embodiments, the central portion of the frame includes multiple struts, and the proximal flaps do not overlap any of the struts.

In some embodiments, the proximal flaps fit between the struts while abutting the struts.

In some embodiments, the apparatus further includes:
an axial shaft configured to rotate;
a radial bearing configured to radially stabilize the axial shaft while the axial shaft rotates; and
a bearing housing, which houses the radial bearing and is coupled to the frame distally to the frame,
the impeller is disposed over the axial shaft, and
the inlet guard is coupled to the bearing housing.

In some embodiments, the inlet guard includes multiple distal flaps, which are coupled to the bearing housing.

In some embodiments,
the distal portion of the frame is frustoconical, and
the main body of the inlet guard is frustoconical and is fixed over the distal portion of the frame.

In some embodiments, the inlet guard includes a rolled sheet of the material.

In some embodiments, the main body of the inlet guard is flat and is disposed within the frame.

In some embodiments, the apparatus further includes an axial shaft configured to rotate,
the impeller is disposed over the axial shaft, and
the main body of the inlet guard is perpendicular to the axial shaft.

There is further provided, in accordance with some embodiments, an apparatus including a blood pump. The blood pump includes a frame including a proximal portion, a central portion including multiple struts, and a distal portion. The blood pump further includes an impeller, which is configured to pump blood of a subject proximally, disposed within the frame. The blood pump further includes a pump-outlet tube, which is fixed over the proximal portion of the frame and at least part of the central portion of the frame. The blood pump further includes an inlet guard, which is distal to the impeller, includes a main body shaped to define one or more blood-inlet openings configured to allow passage of the blood therethrough, and includes multiple proximal flaps, which are coupled to the pump-outlet tube and are fixed at least partly over the central portion of the frame without overlapping any of the struts.

In some embodiments, the proximal flaps fit between the struts while abutting the struts.

In some embodiments, the pump-outlet tube is configured to traverse an aortic valve of the subject, and the impeller is configured to pump the blood from a left ventricle of a heart of the subject, through the pump-outlet tube, into an aorta of the subject.

In some embodiments, the blood-inlet openings are sized such that the inlet guard is configured to block chordae tendineae, trabeculae carneae, and papillary muscles of a left ventricle of a heart of the subject from entering the frame.

In some embodiments, for each of the blood-inlet openings, a span of the blood-inlet opening in at least one direction is less than 1 mm.

In some embodiments, an area of each of the blood-inlet openings is 0.05-5 mm².

In some embodiments, a porosity of the inlet guard is at least 40 percent.

In some embodiments, each of the blood-inlet openings is hexagonal.

In some embodiments, a distance between each pair of adjacent ones of the blood-inlet openings is 0.01-0.1 mm.

In some embodiments, the inlet guard is coupled to the distal portion of the frame.

In some embodiments, the apparatus further includes an inner lining that lines at least part of the central portion of the frame and is coupled to the inlet guard.

In some embodiments, the proximal flaps are coupled to the inner lining.

In some embodiments, the pump-outlet tube and the inner lining are heat welded to one another with the proximal flaps being disposed between the pump-outlet tube and the inner lining.

In some embodiments, the proximal flaps are shaped to define multiple flap openings, and the pump-outlet tube and the inner lining are heat welded to one another at least partly via the flap openings.

In some embodiments, a glass-transition temperature of the inlet guard is higher than the respective glass-transition temperatures of each of the inner lining and the pump-outlet tube.

In some embodiments,
the inner lining is made of a polyurethane,
the pump-outlet tube is made of a polyether block amide, and
the inlet guard is made of a polyether ether ketone.

In some embodiments,
the inner lining and the pump-outlet tube are made of the same type or different types of polyurethane, and
the inlet guard is made of a polyether ether ketone.

In some embodiments, the apparatus further includes:
an axial shaft configured to rotate;
a radial bearing configured to radially stabilize the axial shaft while the axial shaft rotates; and
a bearing housing, which houses the radial bearing and is coupled to the frame distally to the frame,
the impeller is disposed over the axial shaft, and
the inlet guard is coupled to the bearing housing.

In some embodiments, the inlet guard includes multiple distal flaps, which are coupled to the bearing housing.

In some embodiments,
the distal portion of the frame is frustoconical, and
the main body of the inlet guard is frustoconical and is fixed over the distal portion of the frame.

In some embodiments, the inlet guard includes a rolled sheet of material.

In some embodiments, the main body of the inlet guard is flat and is disposed within the frame.

In some embodiments, the apparatus further includes an axial shaft configured to rotate,
the impeller is disposed over the axial shaft, and
the main body of the inlet guard is perpendicular to the axial shaft.

There is further provided, in accordance with some embodiments, apparatus including:
a blood pump, including:
a frame;
an inner lining, which has a flexural modulus of between 0.1GPa and 2 GPa and lines at least part of the frame;
an impeller, which is configured to pump blood of a subject, disposed within the frame; and
a pump-outlet tube, which has a flexural modulus of between 0.1 GPa and 0.8 GPa, is fixed over at least part of the frame, is heat welded to the inner lining,
   respective melting temperatures of the inner lining and the pump-outlet tube being within 20 °C of one another.

In some embodiments, the inner lining and the pump-outlet tube are made of the same material, such that the melting temperatures are the same as one another.

In some embodiments, the flexural modulus of the inner lining is between 0.1 GPa and 0.8 GPa.

In some embodiments, the flexural modulus of the inner lining is between 0.8 GPa and 2 GPa.

In some embodiments, the flexural modulus of the pump-outlet tube is between 0.1 GPa and 0.5 GPa.

In some embodiments, the inner lining is made of a first polymer and the pump-outlet tube is made of a second polymer and the first and second polymers belong to a single class of polymer.

In some embodiments, the first polymer and the second polymer are the same as each other.

In some embodiments, the first polymer and the second polymer are different polymers from each other.

In some embodiments, the first and second polymers are both polyurethanes.

In some embodiments, the respective melting temperatures of the inner lining and the pump-outlet tube are within 10 °C of one another.

In some embodiments, the respective melting temperatures of the inner lining and the pump-outlet tube are within 5 °C of one another.

In some embodiments, the respective melting temperatures of the inner lining and the pump-outlet tube are the same as one another.

In some embodiments, respective glass-transition temperatures of the inner lining and the pump-outlet tube are within 20 °C of one another.

In some embodiments, the respective glass-transition temperatures of the inner lining and the pump-outlet tube are within 10 °C of one another.

In some embodiments, the respective glass-transition temperatures of the inner lining and the pump-outlet tube are within 5 °C of one another.

In some embodiments, the respective glass-transition temperatures of the inner lining and the pump-outlet tube are the same as one another.

There is further provided, in accordance with some embodiments, including:
a blood pump, including:
a frame;
an inner lining, made of a first polymer that has a flexural modulus of between 0.1GPa and 2 GPa and lines at least part of the frame;
an impeller, which is configured to pump blood of a subject, disposed within the frame; and
a pump-outlet tube, which is made of a second polymer that has a flexural modulus of between 0.1 GPa and 0.8 GPa, is fixed over at least part of the frame, is heat welded to the inner lining,
   the first and second polymers belong to a single class of polymer.

In some embodiments, the flexural modulus of the inner lining is between 0.1 GPa and 0.8 GPa.

In some embodiments, the flexural modulus of the inner lining is between 0.8 GPa and 2 GPa.

In some embodiments, the flexural modulus of the pump-outlet tube is between 0.1 GPa and 0.5 GPa.

In some embodiments, the first polymer and the second polymer are the same as each other.

In some embodiments, the first polymer and the second polymer are different polymers from each other.

In some embodiments, the first and second polymers are both polyurethanes.

In some embodiments, the respective melting temperatures of the inner lining and the pump-outlet tube are within 20 °C of one another.

In some embodiments, the respective melting temperatures of the inner lining and the pump-outlet tube are within 10 °C of one another.

In some embodiments, the respective melting temperatures of the inner lining and the pump-outlet tube are within 5 °C of one another.

In some embodiments, the respective melting temperatures of the inner lining and the pump-outlet tube are the same as one another.

In some embodiments, respective glass-transition temperatures of the inner lining and the pump-outlet tube are within 20 °C of one another.

In some embodiments, the respective glass-transition temperatures of the inner lining and the pump-outlet tube are within 10 °C of one another.

In some embodiments, the respective glass-transition temperatures of the inner lining and the pump-outlet tube are within 5 °C of one another.

In some embodiments, the respective glass-transition temperatures of the inner lining and the pump-outlet tube are the same as one another.

The present disclosure will be more fully understood from the following detailed description of embodiments thereof, taken together with the drawings, in which:

### BRIEF DESCRIPTION OF THE DRAWINGS

Fig. 1A is a schematic illustration of a ventricular assist system, in accordance with some embodiments;
Fig. 1B schematically illustrates the deployment of a ventricular assist device within the left ventricle of a subject, in accordance with some embodiments;
Figs. 1C and 1D are schematic illustrations of a pump-head portion of a ventricular assist device, in accordance with some embodiments;
Fig. 2 is a schematic illustration of a frame that houses an impeller of a ventricular assist device, in accordance with some embodiments;
Figs. 3A and 3B are schematic illustrations of an impeller of a ventricular assist device, in accordance with some embodiments;
Fig. 4 is a schematic illustration of an impeller disposed inside the frame of a ventricular assist device, in accordance with some embodiments;
Figs. 5A and 5B are schematic illustrations of the impeller and the frame of the ventricular assist device, respectively in non-radially-constrained and radially-constrained states thereof, in accordance with some embodiments;
Fig. 5C is an enlarged schematic illustration of the proximal end of the frame of the ventricular assist device, in accordance with some embodiments;
Fig. 5D, 5E, and 5F are schematic illustrations of a coupling element, in accordance with some embodiments;
Figs. 6A, 6B, and 6C are schematic illustrations of a ventricular assist device that includes an inner lining on the inside of the frame that houses the impeller of a ventricular assist device, in accordance with some embodiments;
Figs. 7A, 7B, 7C, and 7D are schematic illustrations of a pump-outlet tube that defines blood-inlet openings at a distal end thereof, in accordance with some embodiments;
Fig. 8A is a schematic illustration of a sheet of material, in accordance with some embodiments;
Fig. 8B is a schematic illustration of a frustoconical inlet guard formed from the sheet shown in Fig. 8A, in accordance with some embodiments;
Fig. 8C is a schematic illustration of a sheet of material, in accordance with some embodiments;
Fig. 8D is a schematic illustration of a frustoconical inlet guard fixed over the distal portion of a frame, in accordance with some embodiments;
Fig. 9A is a schematic illustration of an expandable element surrounding a delivery tube, in accordance with some embodiments;
Fig. 9B is a schematic illustration of a pump-head portion of a ventricular assist device, in accordance with some embodiments;
Figs. 10A, 10B, 10C, and 10D are schematic illustrations of a pump-head portion of a ventricular assist device, in accordance with some embodiments;
Fig. 10E is a schematic illustration of a pump-outlet tube that defines a blood-flow chamber at its proximal end, in accordance with some embodiments;
Fig. 10F is a schematic illustration of an inflatable element disposed at the proximal end of a pump-outlet tube, in accordance with some embodiments;
Fig. 10G is a schematic illustration of an inflatable element disposed proximally to a pump-outlet tube, in accordance with some embodiments;
Fig. 10H is a schematic illustration of a pump-head portion of a ventricular assist device, in accordance with some embodiments;
Figs. 10I and 10J are schematic illustrations of an inflatable element from oblique and frontal perspectives, respectively, in accordance with some embodiments;
Figs. 10K, 10L, 10M, and 10N are schematic illustrations of a pump-head portion of a ventricular assist device, in accordance with some embodiments.
Fig. 10O shows a schematic frontal view of the proximal end of a pump-head portion of a ventricular assist device, in accordance with some embodiments;
Fig. 10P is a schematic illustration of a pump-head portion of a ventricular assist device, in accordance with some embodiments;
Figs. 11A, 11B, 11C, and 11D are schematic illustrations of portions of a ventricular assist device, in accordance with some embodiments;
Fig. 11E is a schematic illustration of an inlet guard, in accordance with some embodiments; and
Fig. 11F is a schematic illustration of an inlet guard disposed within a frame, in accordance with some embodiments.

### DETAILED DESCRIPTION

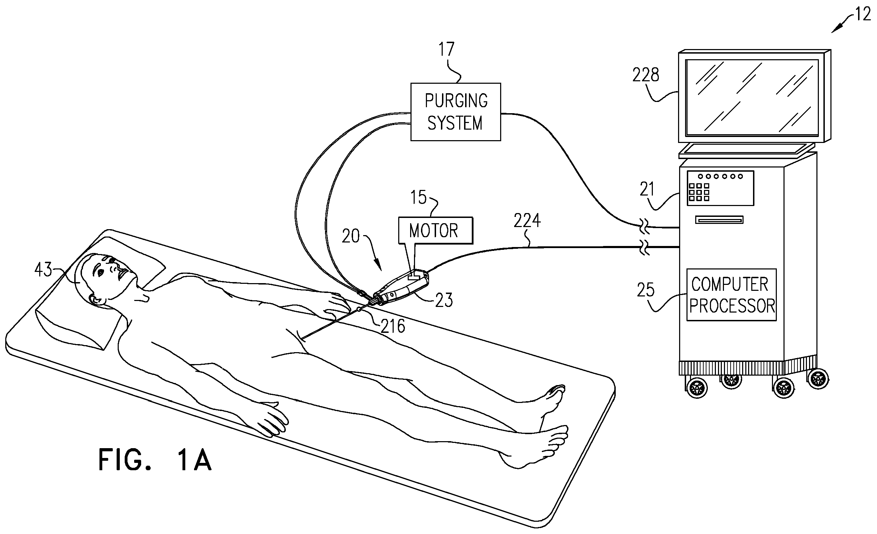

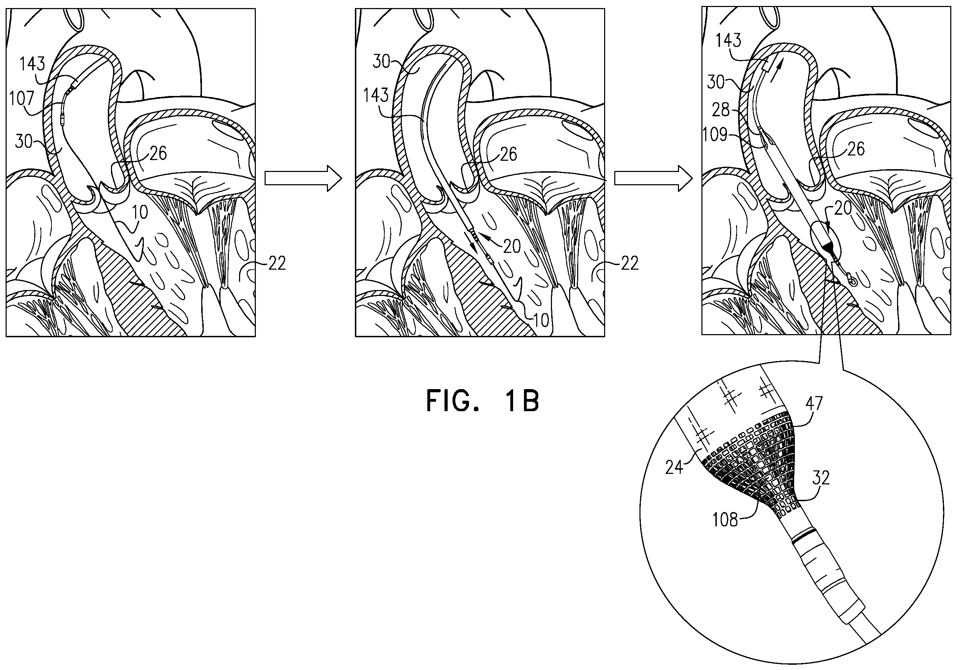

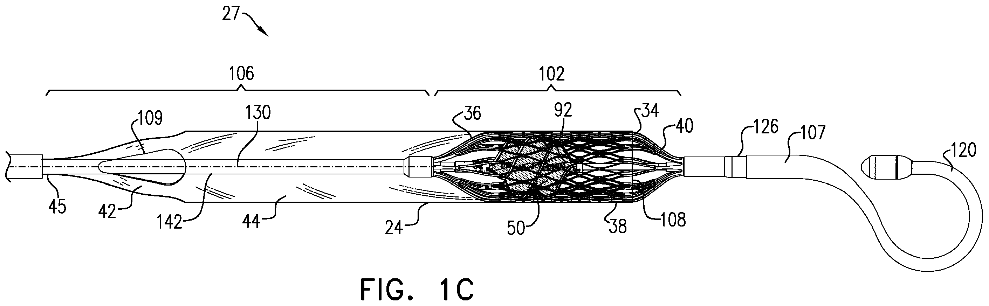

Reference is initially made to Fig. 1A, which is a schematic illustration of a ventricular assist system 12 comprising a ventricular assist device 20 configured to assist left-ventricular function of a subject 43, in accordance with some embodiments. Reference is also made to Fig. 1B, which schematically illustrates the deployment of device 20 within the left ventricle 22 of subject 43, in accordance with some embodiments. Reference is additionally made to Fig. 1C, which is a schematic illustration of a pump-head portion 27 of device 20, in accordance with some embodiments. Given that the scope of the present disclosure includes using the apparatus and methods described herein in anatomical locations other than the left ventricle and the aorta, ventricular assist device 20 and/or portions thereof are sometimes referred to herein (in the specification and the claims) as a blood pump.

Ventricular assist device 20 comprises a pump-outlet tube 24, which is shaped to define one or more blood-outlet openings 109. Typically, a proximal section 106 of the pump-outlet tube defines blood-outlet openings 109 such that the blood-outlet openings are near the proximal end 28 of pump-outlet tube 24. The pump-outlet tube is configured for insertion, through the aorta 30 of subject 43, into left ventricle 22 such that, by virtue of the pump-outlet tube traversing the aortic valve 26 of the subject, blood-outlet openings 109 are disposed within the aorta and a distal section 102 of the pump-outlet tube, which includes the distal end 32 of the pump-outlet tube, is disposed within the left ventricle. Pump-outlet tube 24 (which may also be referred to as a "blood-pump tube") is typically an elongate tube, an axial length of the pump-outlet tube typically being substantially larger than its diameter.

The ventricular assist device further comprises an impeller 50, which in some embodiments is disposed within distal section 102. Impeller 50 is configured to pump blood of the subject proximally through the pump-outlet tube such that the blood exits the pump-outlet tube via blood-outlet openings 109. Thus, during operation of impeller 50, blood flows from the pump-outlet tube into the ascending aorta.

The ventricular assist device further comprises a delivery tube 142 configured to extend, from outside the body of the subject, through the pump-outlet tube to the distal section of the pump-outlet tube. The device further comprises a drive cable 130 passing through the delivery tube and operatively coupled to impeller 50. System 12 further comprises a motor unit 23, which comprises a motor 15 configured to rotate the impeller via drive cable 130.

The pump-outlet tube typically defines one or more blood-inlet openings 108 at the distal end of the pump-outlet tube, via which blood flows into the pump-outlet tube, from the left ventricle, during operation of the impeller. As shown in Fig. 1C, for some applications, the pump-outlet tube defines a single axially-facing blood-inlet opening. Alternatively, the pump-outlet tube defines a plurality of lateral blood-inlet openings, e.g., as shown in Fig. 1B.

For some applications, the ventricular assist device is used to assist the functioning of a subject's left ventricle during a percutaneous coronary intervention. In such cases, the ventricular assist device is typically used for a period of up to six hours (e.g., up to ten hours), during a period in which there is risk of developing hemodynamic instability (e.g., during or immediately following the percutaneous coronary intervention). Alternatively or additionally, the ventricular assist device is used to assist the functioning of a subject's left ventricle for a longer period (e.g., 2-20 days, e.g., 4-14 days) upon a patient suffering from cardiogenic shock, which may include any low-cardiac-output state (e.g., acute myocardial infarction, myocarditis, cardiomyopathy, post-partum, etc.). For some applications, the ventricular assist device is used to assist the functioning of a subject's left ventricle for yet a longer period (e.g., several weeks or months), e.g., in a "bridge to recovery" treatment. For some such applications, the ventricular assist device is permanently or semi-permanently implanted, and the impeller of the ventricular assist device is powered transcutaneously, e.g., using an external antenna that is magnetically coupled to the impeller.

As shown in Fig. 1B, which shows steps in the deployment of the ventricular assist device in the left ventricle, typically the distal end of the ventricular assist device, which comprises pump-outlet tube 24, a distal-tip element 107, and other components described in detail below, is guided to the left ventricle, and inserted into the left ventricle, over a guidewire 10 (e.g., a standard 0.018 inch guidewire), which passes through distal-tip element 107. Typically, guidewire 10 comprises a soft atraumatic distal end. In some embodiments, prior to threading the guidewire through the device, the distal-tip element is straightened using the tip-straightening element described with reference to Figs. 23A-C of WO 21/205346 to Tuval, which is incorporated herein by reference.

During the insertion of the distal end of the device into the left ventricle, a delivery catheter 143 is disposed over the distal end of the device, such that delivery catheter 143 holds pump-outlet tube 24 in a radially-constrained configuration. In some embodiments, the delivery catheter is disposed within a standard sheath, such as a 10 Fr sheath. Once the distal end of the device is disposed in the left ventricle (and the sheath, if used, is withdrawn), the pump-outlet tube, along with other components of the device, are removed from the delivery catheter within the left ventricle, by retracting the delivery catheter from over the device. (In this context, advancing the device without advancing the delivery catheter is also referred to as retraction of the delivery catheter.) The retraction of the delivery catheter typically causes self-expandable components of the distal end of the device, such as the pump-outlet tube, to assume non-radially-constrained configurations, as described in further detail hereinbelow. Subsequently, the delivery catheter is typically retracted to the descending aorta, and guidewire 10 is withdrawn from the subject's body.

Typically, distal-tip element 107 is positioned at the apex of the left ventricle, e.g., as described with reference to Figs. 17B-D of WO 24/057252 to Tuval, which is incorporated herein by reference.

In some embodiments, the positioning of the distal-tip element, along with the withdrawing of the guidewire from the distal-tip element, are performed after the removal of the pump-outlet tube from the delivery catheter. The distal end of the device, when not radially constrained, has greater flexibility, relative to when radially constrained, and this flexibility facilitates positioning the distal-tip element.

In some embodiments, the distal-tip element is positioned at the apex by pushing the distal-tip element toward the apex while withdrawing the guidewire from the distal-tip element. If, on the other hand, the guidewire were to be withdrawn before pushing the distal-tip element toward the apex, it might be challenging to position the distal-tip element correctly. Likewise, if the guidewire were withdrawn after pushing the distal-tip element toward the apex, the withdrawal of the guidewire might cause the distal-tip element to become reshaped, which might also result in incorrect positioning.

Following the removal of the guidewire from the device, driven-magnet unit 310 of the device (shown in Figs. 18A-B, for example) and drive cable 130 are coupled to motor unit 23, and the device is activated.

For some applications, in order to withdraw the left ventricular device from the subject's body at the end of the treatment, the delivery catheter is advanced over the distal end of the device, which causes the self-expandable components of the distal end of the device (e.g., the pump-outlet tube) to assume radially-constrained configurations. Alternatively or additionally, the distal end of the device is retracted into the delivery catheter which causes the self-expandable components of the distal end of the device to assume radially-constrained configurations.

In some embodiments, the distal end of the device is reinserted into the delivery catheter within the descending aorta of the subject. An advantage of performing the reinsertion in the descending aorta - rather than, for example, the left ventricle, ascending aorta, or aortic arch - is that the descending aorta is straight. Moreover, in some cases, the device might release thrombi or debris as the device is reinserted. In the descending aorta, there is less risk of this thrombi or debris reaching the brain.

For some applications (not shown), the ventricular assist device and/or delivery catheter 143 includes an ultrasound transducer at its distal end and the ventricular assist device is advanced toward the subject's ventricle under ultrasound guidance.

System 12 further comprises a control console 21, which comprises a computer processor 25 configured to drive the impeller to rotate. For example, via a cable 224, the computer processor may control motor 15, which, as described above, drives the impeller to rotate via drive cable 130. For some applications, the computer processor is configured to detect or estimate a physiological parameter of the subject (such as left-ventricular pressure, native cardiac output, cardiac afterload, rate of change of left-ventricular pressure, etc.) and to control rotation of the impeller in response thereto. Typically, the operations described herein that are performed by the computer processor, transform the physical state of a memory, which is a real physical article that is in communication with the computer processor, to have a different magnetic polarity, electrical charge, or the like, depending on the technology of the memory that is used. Computer processor 25 is typically a hardware device programmed with computer program instructions to produce a special-purpose computer. For example, when programmed to perform the techniques described herein, computer processor 25 typically acts as a special-purpose, ventricular-assist computer processor and/or a special-purpose, blood-pump computer processor.

For some applications, a purging system 17 (shown in Fig. 1A) drives a fluid (e.g., a glucose solution) to pass through portions of ventricular assist device 20, for example, in order to cool portions of the device, to purge and/or lubricate interfaces between rotating parts and stationary bearings, and/or in order to wash debris from portions of the device.

Typically, console 21 further comprises a display 228, embodiments of which are described below with reference to Fig. 14.

Typically, along distal section 102 of pump-outlet tube 24, a frame 34 is disposed at least partly within the pump-outlet tube and around impeller 50, distal-tip element 107 being disposed distally with respect to frame 34. The frame is typically made of a shape-memory alloy, such as nitinol. For some applications, the shape-memory alloy of the frame is shape set such that at least a portion of the frame (and thereby distal section 102 of tube 24) assumes a generally circular, elliptical, or polygonal cross-sectional shape in the absence of any forces being applied to distal section 102 of tube 24. By assuming its generally circular, elliptical, or polygonal cross-sectional shape, the frame is configured to hold the distal section of the pump-outlet tube in an open state. Typically, during operation of the ventricular assist device, the distal section of the pump-outlet tube is configured to be placed within the subject's body such that the distal section of the pump-outlet tube is disposed at least partially within the left ventricle.

For some applications, along proximal section 106 of pump-outlet tube 24, the frame is not disposed within the pump-outlet tube, and the pump-outlet tube is therefore not supported in an open state by frame 34. Pump-outlet tube 24 is typically made of a blood-impermeable collapsible material, such that the pump-outlet tube is collapsible. For example, pump-outlet tube 24 may include a polyurethane, polyester, and/or silicone. Alternatively or additionally, the pump-outlet tube is made of polyethylene terephthalate (PET) and/or polyether block amide (e.g., PEBAX^{®}). For some applications (not shown), the pump-outlet tube is reinforced with a reinforcement structure, e.g., a braided reinforcement structure, such as a braided nitinol tube. Typically, the proximal section of the pump-outlet tube is configured to be placed such that it is at least partially disposed within the subject's ascending aorta. For some applications, the proximal section of the pump-outlet tube traverses the subject's aortic valve, passing from the subject's left ventricle into the subject's ascending aorta, as shown in Fig. 1B.

As described hereinabove, the pump-outlet tube typically defines one or more blood-inlet openings 108 at the distal end of the pump-outlet tube, via which blood flows into the pump-outlet tube from the left ventricle, during operation of the impeller. For some applications, the proximal section of the pump-outlet tube defines one or more blood-outlet openings 109, via which blood flows from the pump-outlet tube into the ascending aorta during operation of the impeller. Typically, the pump-outlet tube defines a plurality of blood-outlet openings 109, for example, between two and eight blood-outlet openings (e.g., between two and four blood-outlet openings). During operation of the impeller, the pressure of the blood flow through the pump-outlet tube typically maintains the proximal section of the tube in an open state. For some applications, in the event that, for example, the impeller malfunctions, the proximal section of the pump-outlet tube is configured to collapse inwardly, in response to pressure outside of the proximal section of the pump-outlet tube exceeding pressure inside the proximal section of the pump-outlet tube. In this manner, the proximal section of the pump-outlet tube acts as a safety valve, preventing retrograde blood flow into the left ventricle from the aorta.

Referring again to Fig. 1C, for some applications, frame 34 is shaped such that the frame defines a proximal conical (or "frustoconical") portion 36, a central cylindrical portion 38, and a distal conical portion 40. Typically, the proximal conical portion is proximally-facing, i.e., facing such that the narrow end of the cone is proximal with respect to the wide end of the cone. Further typically, the distal conical portion is distally-facing, i.e., facing such that the narrow end of the cone is distal with respect to the wide end of the cone.

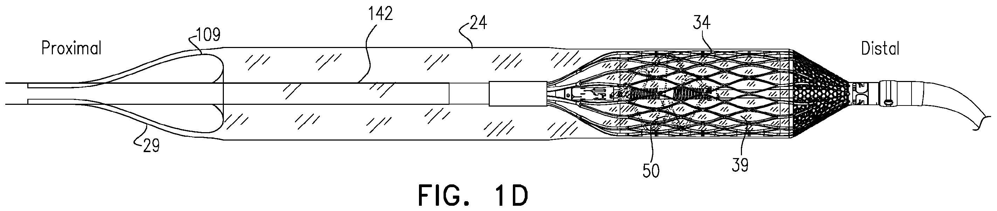

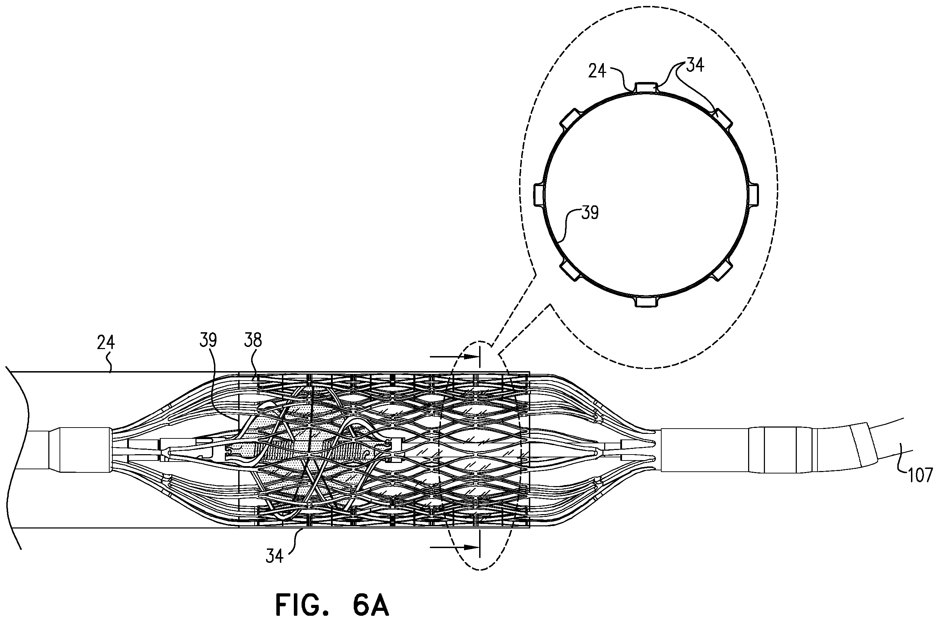

For some applications, within at least a portion of frame 34 (e.g., along all of, or a portion of, the central cylindrical portion of the frame), an inner lining 39, shown in Fig. 1D for example, lines the frame. In accordance with respective applications, inner lining 39 partially overlaps or fully overlaps pump-outlet tube 24 over the portion of the frame that the inner lining lines, as described in further detail hereinbelow with reference to Figs. 6A-B. For other applications, as shown in Fig. 1C, the pump-head portion does not comprise inner lining 39.

In some embodiments, pump-outlet tube 24 includes a conical proximal portion 42 and a cylindrical central portion 44, which typically spans proximal section 106 and distal section 102. The proximal conical portion is typically proximally-facing, i.e., facing such that the narrow end of the cone is proximal with respect to the wide end of the cone. Typically, blood-outlet openings 109 are defined by pump-outlet tube 24 such that the openings extend at least partially along the proximal conical portion of tube 24. For some such applications, the blood-outlet openings are teardrop-shaped, as shown in Fig. 1C. Typically, the teardrop-shaped nature of the blood-outlet openings in combination with the openings extending at least partially along the proximal conical portion of tube 24 causes blood to flow out of the blood-outlet openings along flow lines that are substantially parallel with the longitudinal axis of tube 24 at the location of the blood-outlet openings.

For some applications (not shown), the diameter of pump-outlet tube 24 changes along the length of the central portion of the pump-outlet tube, such that the central portion of the pump-outlet tube has a frustoconical shape. For example, the central portion of the pump-outlet tube may widen from its proximal end to its distal end, or may narrow from its proximal end to its distal end. For some applications, at its proximal end, the central portion of the pump-outlet tube has a diameter of between 5 and 7 mm, and at its distal end, the central portion of the pump-outlet tube has a diameter of between 8 and 12 mm.

In some embodiments, drive cable 130 is coupled to an axial shaft 92, which passes through impeller 50 and is configured to rotate the impeller. In some such embodiments, distal-tip element 107 comprises an axial-shaft-receiving tube 126 and a distal-tip portion 120. Axial-shaft-receiving tube 126 is configured to receive a distal portion of axial shaft 92 during axial back-and-forth motion of the axial shaft, and/or during delivery of the ventricular assist device. (Typically, during delivery of the ventricular assist device, the frame is maintained in a radially-constrained configuration, which typically causes the axial shaft to be disposed in a different position with respect to the frame relative to its disposition with respect to the frame during operation of the ventricular assist device.) Typically, distal-tip portion 120 is configured to assume a curved shape upon being deployed within the subject's left ventricle, e.g., as shown in Fig. 1C. For some applications, the curvature of the distal-tip portion is configured to provide an atraumatic tip to ventricular assist device 20. Alternatively or additionally, the distal-tip portion is configured to space blood-inlet openings 108 of the ventricular assist device from walls of the left ventricle.

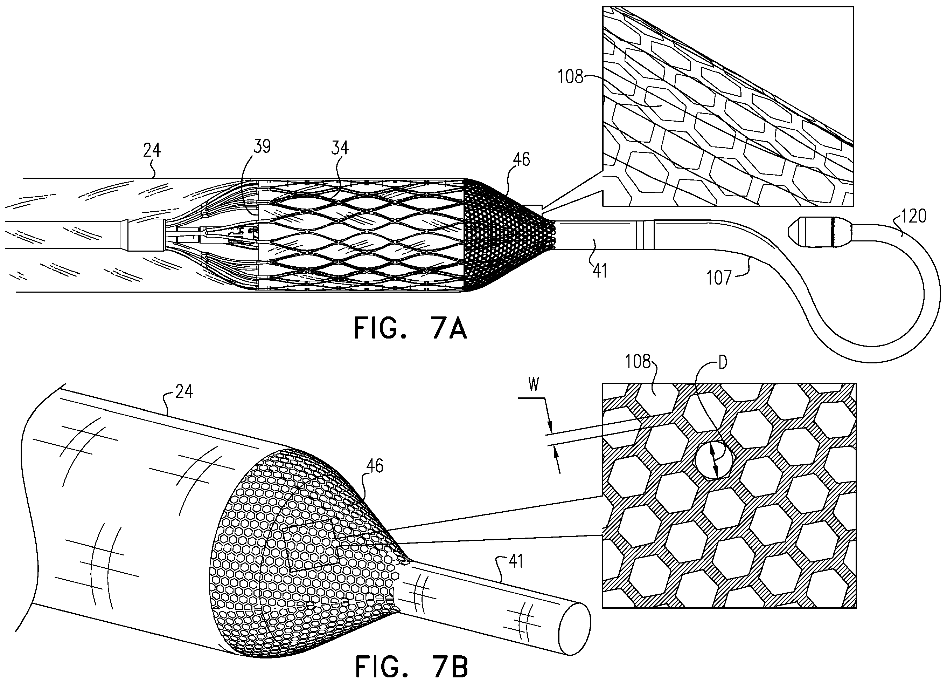

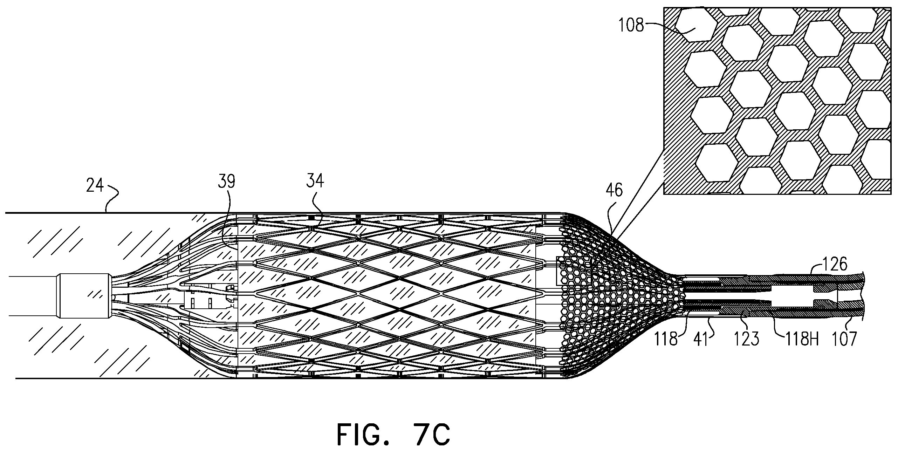

As shown in the enlarged portion of Fig. 1B, for some applications, pump-outlet tube 24 extends to the end of distal conical portion 40 of the frame, and the pump-outlet tube defines a plurality of lateral blood-inlet openings 108, as described in further detail hereinbelow. For some such applications, the pump-outlet tube defines a distal conical (or "frustoconical") portion 47 that is distally facing, i.e., facing such that the narrow end of the cone is distal with respect to the wide end of the cone. For some such applications (not shown), the pump-outlet tube defines two to four lateral blood-inlet openings (e.g., four lateral blood-inlet openings). Typically, for such applications, each of the blood-inlet openings defines an area of more than 20 square mm (e.g., more than 30 square mm), and/or less than 60 square mm (e.g., less than 50 square mm), e.g., 20-60 square mm, or 30-50 square mm. Alternatively or additionally, the outlet tube defines a greater number of smaller lateral blood-inlet openings, e.g., more than 10 blood-inlet openings, more than 100 blood-inlet openings, more than 200 blood-inlet openings, or more than 300 blood-inlet openings, e.g., 50-100 blood-inlet openings, 100-300 blood-inlet openings, or 300-500 blood-inlet openings. For some such applications, each of the blood-inlet openings defines an area of more than 0.05 square mm (e.g., more than 0.1 square mm), and/or less than 3 square mm (e.g., less than 1 square mm), e.g., 0.05-3 square mm, or 0.1-1 square mm. Alternatively, each of the blood-inlet openings defines an area of more than 0.1 square mm (e.g., more than 0.3 square mm), and/or less than 5 square mm (e.g., less than 1 square mm), e.g., 0.1-5 square mm, or 0.3-1 square mm. Such applications are described in further detail hereinbelow, for example, with reference to Figs. 7A-D.

As described above, blood-inlet openings 108 are, in some embodiments, defined by distal conical portion 47 of the pump-outlet tube. As such, even the blood-inlet openings that are described as "lateral blood-inlet openings" are not necessarily oriented entirely laterally with respect to the longitudinal axis of the pump-outlet tube. Rather, they are, in some embodiments, obliquely disposed with respect to the longitudinal axis of the pump-outlet tube. By contrast, in some embodiments, the blood-outlet openings are described as "laterally-facing blood-outlet openings" because in such embodiments the blood-outlet openings are disposed laterally with respect to the longitudinal axis of the pump-outlet tube, by virtue of being defined by the central cylindrical portion of the pump-outlet tube. (In other embodiments, the blood-outlet openings are disposed obliquely with respect to the longitudinal axis of the pump-outlet tube, by virtue of being defined at least partially by the proximal conical portion of the pump-outlet tube.)

In some embodiments, proximally to proximal conical portion 42, the pump-outlet tube defines a tubular coupling portion 45, via which the pump-outlet tube is coupled (e.g., via an adhesive) to delivery tube 142. For some such embodiments, the pump-outlet tube is manufactured from a single continuous tube, with respective portions of the tube being molded to define tubular coupling portion 45, proximal conical portion 42, distal conical portion 47, and cylindrical central portion 44. Typically, in such cases, the blood-inlet openings and the blood-outlet openings are cut (e.g., laser cut) from the tube. In some embodiments, before adhering the tubular coupling portion to delivery tube 142 of the ventricular assist device, the tubular coupling portion is cut (e.g., in a tapered manner), so as to reduce the thickness of the layer of the pump-outlet tube that is coupled to delivery tube 142 and/or to prevent folds forming in the tubular coupling portion of the pump-outlet tube.

In some embodiments, (a) blood-outlet openings 109 are defined by portions of the wall of the blood outlet tube that at least partially extends into the proximal conical portion of the pump-outlet tube, and/or (b) blood-outlet openings 109 are laterally facing, by virtue of being defined by the central cylindrical portion of pump-outlet tube 24. The scope of the present disclosure includes combining other features of the pump-outlet tube and/or other portions of the ventricular assist device with any configuration of blood-outlet openings that are described and/or shown in the present application.

It is noted that the above description of pump-outlet tube 24 and blood-outlet openings 109 is also applicable to other embodiments described herein. Furthermore, the scope of the present disclosure includes combining a pump-outlet-tube that defines a single axially-facing blood-inlet opening 108 as shown in Fig. 1C, or a pump-outlet-tube that defines a plurality of lateral blood-inlet openings 108 as shown in Fig. 1B, with other features of the ventricular assist device that are described herein, *mutatis mutandis.*

In some embodiments, a pressure sensor 216 measures the pressure of blood in the subject's left ventricle, e.g., as described in WO 24/057252 to Tuval, which is incorporated herein by reference.

Reference is now made to Fig. 1D, which is a schematic illustration of a pump-head portion of a ventricular assist device, in accordance with some embodiments.

In some embodiments, pump-outlet tube 24 does not define a tubular coupling portion. Rather, initially, the proximal portion of the tube that will form the proximal conical section is shaped as a cylinder (which is typically continuous with the cylinder shape of the central portion). From this proximal portion of the tube, strips are cut (e.g., laser cut), leaving other strips 29 still attached to, and extending proximally from, the central cylindrical portion of the tube. The proximal ends of strips 29 are then adhered to delivery tube 142 of the ventricular assist device, in such a manner that they define a proximal conical portion of the pump-outlet tube that defines blood-outlet openings 109. In other words, blood-outlet openings 109 are formed between strips 29, by adhering the strips to delivery tube 142 of the ventricular assist device.

For some applications, by forming the proximal conical portion of the pump-outlet tube and the blood-outlet openings using the latter method, the thickness of the layer of the pump-outlet tube that is coupled to delivery tube 142 is less than the thickness of the tubular coupling portion as formed by the former method. For some applications, this reduces the sharpness of the diameter change at the interface between delivery tube 142 and the region at which the proximal end of the pump-outlet tube is coupled to the delivery tube.

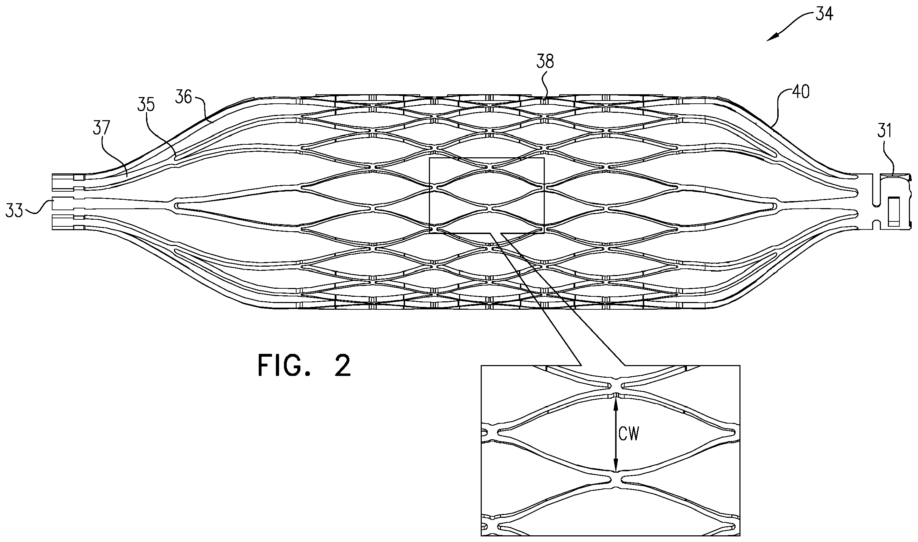

Reference is now made to Fig. 2, which is schematic illustration of frame 34 that houses an impeller of ventricular assist device 20, in accordance with some embodiments. Frame 34 is typically made of a shape-memory alloy, such as nitinol, and the shape-memory alloy of the frame is shape set such that the central portion 38 of the frame (and thereby tube 24) assumes a generally circular, elliptical, or polygonal cross-sectional shape in the absence of any forces being applied to pump-outlet tube 24. By assuming its generally circular, elliptical, or polygonal cross-sectional shape, the frame is configured to hold the distal portion of the tube in an open state. (Given that, typically, central portion 38 of the frame has a circular cross-section, the central portion of the frame is also referred to herein as the "cylindrical portion" of the frame.)

Typically, the frame is a stent-like frame, in that it comprises struts 37 that, in turn, define cells. In some embodiments, the frame is laser cut from a metal or alloy tube. Typically, the frame is covered with pump-outlet tube 24, and/or covered with an inner lining 39, described hereinbelow with reference to Figs. 6A-B. As described hereinbelow, for some applications, impeller 50 undergoes axial back-and-forth motion with respect to frame 34. Typically, over the course of the motion of the impeller with respect to the frame, the location of the portion of the impeller that defines the maximum span of the impeller is disposed within central cylindrical portion 38 of frame 34. In some cases, if the cells of the central cylindrical portion 38 of frame 34 are too large, then pump-outlet tube 24, and/or inner lining 39 (Fig. 1D), gets stretched between edges of the cells, such that the pump-outlet tube 24, and/or inner lining 39, does not define a circular cross-section. For some applications, if this occurs in the region in which the portion of the impeller that defines the maximum span of the impeller is disposed, this results in a substantially non-constant gap between the edges of the impeller blades and tube 24 (and/or inner lining) at that location, over the course of a rotation cycle of the impeller. For some applications, this may lead to increased hemolysis relative to if there were a substantially constant gap between the edges of the impeller blades and tube 24 (and/or inner lining) at that location, over the course of the rotation cycle of the impeller.

Referring to Fig. 2, at least partially in view of the issues described in the above paragraph, within central cylindrical portion 38 of frame 34, the frame defines a large number of relatively small cells. Typically, when the frame is disposed in its non-radially-constrained configuration, the maximum cell width CW of the each of the cells (i.e., the distance from the inner edge of the strut at the central junction on one side of the cell to the inner edge of the strut at the central junction on the other side of the cell, as measured around the circumference of cylindrical portion 38) within the cylindrical portion of the frame is less than 2 mm, e.g., between 1.4 mm and 1.6 mm, or between 1.6 and 1.8 mm. Since the cells are relatively small, inner lining 39 defines a substantially circular cross-section within the cylindrical portion of the frame.

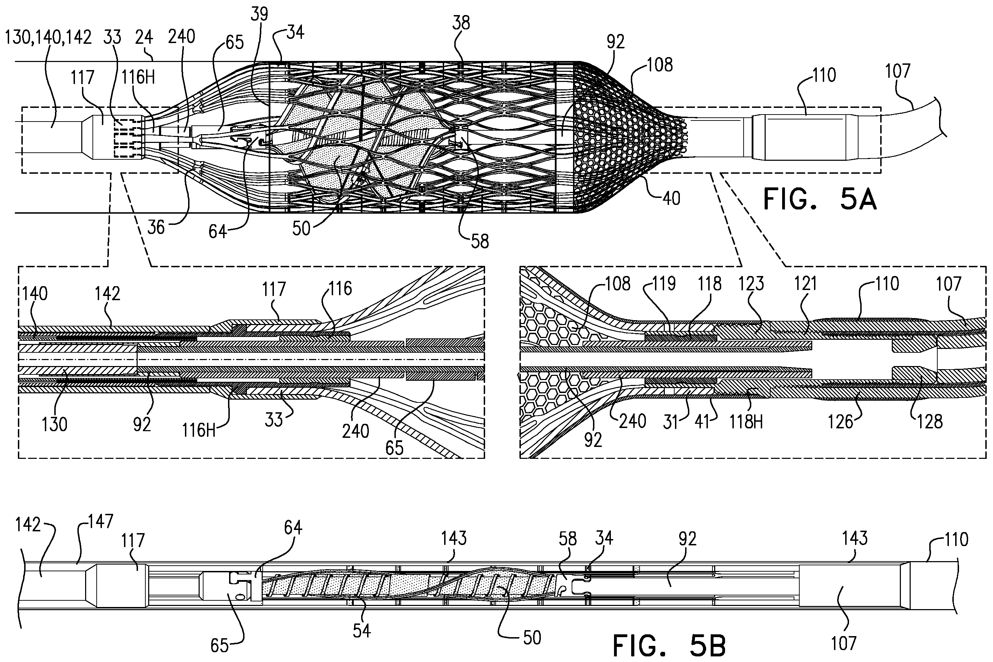

Still referring to Fig. 2, and starting from the distal end of the frame (which is to the right of the figure), typically the frame defines the following portions: (a) coupling portion 31 via which the frame is coupled to a distal bearing housing 118H (shown in Fig. 5A) of the ventricular assist device, (b) distal conical portion 40, (c) central cylindrical portion 38, (d) proximal conical portion 36, and (e) proximal strut junctions 33. As illustrated, as the frame transitions from a proximal end of the frame toward the center of the frame (e.g., as the frame transitions from proximal strut junctions 33, through proximal conical portion 36, and to central cylindrical portion 38), struts 37 of the frame pass through junctions 35, at which the two struts branch from a single strut, in a Y-shape.

During the assembly of the ventricular assist device, the impeller is inserted into frame 34, typically via the open proximal end of the frame. In some embodiments, prior to the insertion of the impeller, pump-outlet tube 24 (Fig. 1C) is fixed over the frame, including over the distal end of the frame. In such embodiments, the impeller cannot be inserted via the distal end of the frame, since the distal end of the frame is covered by pump-outlet tube 24. Therefore, proximal strut junctions 33 are maintained in open states, in order for the impeller to be placed within the frame via the proximal end of the frame. Subsequently to the impeller being inserted via the proximal end of the frame, the proximal strut junctions are closed. For some applications, the proximal strut junctions are closed around the outside of a proximal bearing housing 116H (shown in Fig. 5A), as described in further detail hereinbelow with reference to Figs. 5A-B. Typically, a securing element 117 (e.g., a ring shown in Fig. 5A) holds the strut junctions in their closed configurations around the outside of proximal bearing housing 116H.

In other embodiments, the pump-outlet tube does not extend to the distal end of frame 34, or the distal portion of the pump-outlet tube is fixed over the distal portion of the frame only after the impeller has been inserted into the frame. In some such embodiments, the impeller is inserted into the frame via the distal end of the frame.

In some embodiments, prior to or subsequently to the insertion of the impeller, distal coupling portion 31 is coupled to a distal bearing housing 118H (shown in Fig. 5A), e.g., via a snap-fit mechanism.

Typically, when disposed in its non-radially constrained configuration, frame 34 has a total length of more than 25 mm (e.g., more than 30 mm), and/or less than 50 mm (e.g., less than 45 mm), e.g., 25-50 mm, or 30-45 mm. Typically, when disposed in its radially-constrained configuration (within delivery catheter 143), the length of the frame increases by between 2 and 5 mm. Typically, when disposed in its non-radially constrained configuration, the central cylindrical portion of frame 34 has a length of more than 12 mm (e.g., more than 15 mm), and/or less than 28 mm (e.g., less than 24 mm), e.g., 12-28 mm, or 15-24 mm. For some applications, a ratio of the length of the central cylindrical portion of the frame to the total length of the frame is more than 1:3 and/or less than 3:4, e.g., between 1:3 and 3:4.

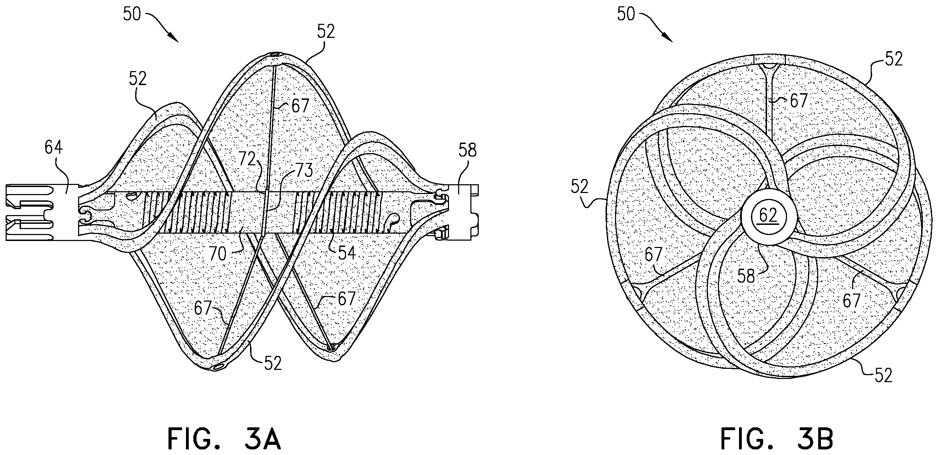

Reference is now made to Figs. 3A-B, which are schematic illustrations of impeller 50, in accordance with some embodiments. Typically, the impeller includes at least one outer helical elongate element 52, which winds around a central axial spring 54, such that the helix defined by the helical elongate element is coaxial with the central axial spring. Typically, the impeller includes two or more helical elongate elements (e.g., three helical elongate elements, as shown in Figs. 3A-B). For some applications, the helical elongate elements and the central axial spring are made of a shape-memory material, e.g., a shape-memory alloy, such as nitinol. Typically, each of the helical elongate elements and the central axial spring support a film 56 of a material (e.g., an elastomer, such as a polyurethane, and/or silicone) therebetween. For some applications, the film of material includes pieces of nitinol embedded therein, for example in order to strengthen the film of material.

Each of the helical elongate elements, together with the film extending from the helical elongate element to the spring, defines a respective impeller blade, with the helical elongate elements defining the outer edges of the blades, and the axial spring defining the axis of the impeller. Typically, the film of material extends along and coats the spring.

Typically, proximal ends of spring 54 and helical elongate elements 52 extend from a proximal bushing (i.e., sleeve bearing) 64 of the impeller, such that the proximal ends of spring 54 and helical elongate elements 52 are disposed at a similar radial distance from the longitudinal axis of the impeller, as each other. Similarly, typically, distal ends of spring 54 and helical elongate elements 52 extend from a distal bushing 58 of the impeller, such that the distal ends of spring 54 and helical elongate elements 52 are disposed at a similar radial distance from the longitudinal axis of the impeller, as each other. The helical elongate elements typically rise gradually from the proximal bushing before reaching a maximum span and then falling gradually toward the distal bushing. Typically, the helical elongate elements are symmetrical along their lengths, such that the rising portions of their lengths are symmetrical with respect to the falling portions of their lengths. Typically, the impeller defines a lumen 62 therethrough, with the lumen typically extending through, and being defined by, spring 54, as well as proximal bushing 64 and distal bushing 58, of the impeller.

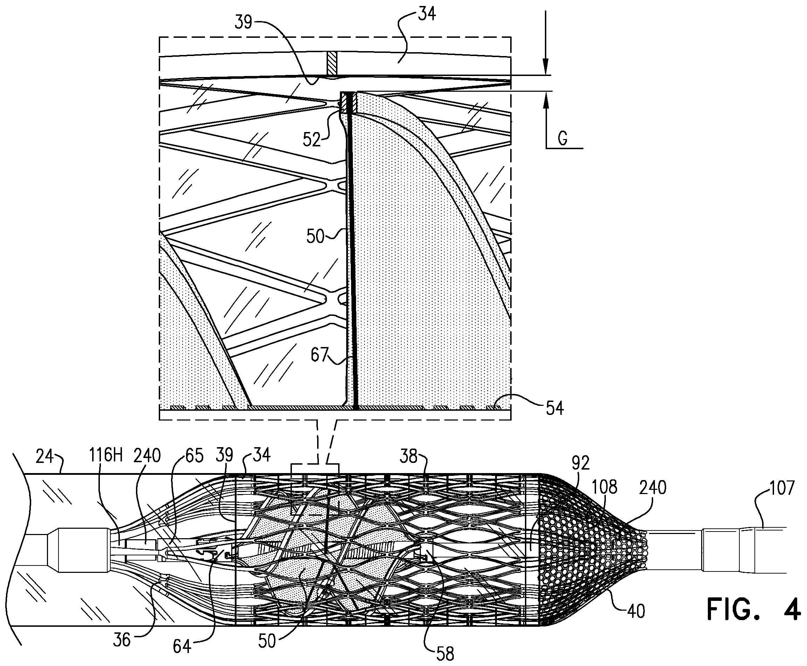

Reference is now made to Fig. 4, which is a schematic illustration of impeller 50 disposed inside frame 34 of ventricular assist device 20, in accordance with some embodiments. For some applications, within at least a portion of frame 34 (e.g., along all of, or a portion of, central cylindrical portion 38 of the frame), inner lining 39 lines the frame. In accordance with respective applications, the inner lining partially overlaps or fully overlaps with pump-outlet tube 24 over the portion of the frame that the inner lining lines, as described in further detail hereinbelow with reference to Figs. 6A-B.

As shown in Fig. 4, typically there is a gap G between the outer edge of impeller 50 and inner lining 39, even at a location at which the span of the impeller is at its maximum. For some applications, it is desirable that the gap between the outer edge of the blade of the impeller and inner lining 39 be relatively small, in order for the impeller to efficiently pump blood from the subject's left ventricle into the subject's aorta. (It is noted that, by virtue of the relatively small gap between the outer edge of impeller 50 and inner lining 39 even at a location at which the span of the impeller is at its maximum, as well as the shape of the impeller, the impeller functions as an axial-flow impeller, with the impeller pumping blood in the axial direction from a distal end of pump-outlet tube 24 to the proximal end of the pump-outlet tube.) It is also desirable that a gap between the outer edge of the blade of the impeller and the inner surface of frame 34 be maintained throughout the rotation of the impeller within frame 34, for example, in order to reduce the risk of hemolysis.

For some applications, when impeller 50 and frame 34 are both disposed in non-radially-constrained configurations and prior to operation of the impeller, gap G between the outer edge of the impeller and the inner lining 39, at the location at which the span of the impeller is at its maximum, is greater than 0.05 mm (e.g., greater than 0.1 mm), and/or less than 1 mm (e.g., less than 0.4 mm), e.g., 0.05-1 mm, or 0.1-0.4 mm.

Typically, an axial shaft 92 passes through the axis of impeller 50, via lumen 62 of the impeller. For some applications, the axial shaft is rigid, e.g., a rigid tube. For some applications, the axial shaft is made of a shape-memory material (e.g., a shape memory alloy, such as nitinol). Typically, such materials have some elasticity, such that in the event that the axial shaft becomes bent (e.g., during delivery of the pump head to the left ventricle), the axial shaft still assumes a straight shape, once deployed inside the subject's body.

Proximal bushing 64 is disposed over axial shaft 92, and distal bushing 58 is disposed over the axial shaft distally from the proximal bushing. For some applications, proximal bushing 64 of the impeller is coupled to the shaft such that the axial position of the proximal bushing with respect to the shaft is fixed, and distal bushing 58 of the impeller is slidable with respect to (i.e., is slidable along) the shaft. For example, the proximal bushing may be coupled to a coupling element 65 disposed on the axial shaft (shown in Fig. 4), for example via a snap-fit mechanism. Alternatively, distal bushing 58 of the impeller is coupled to the shaft such that the axial position of the distal bushing with respect to the shaft is fixed, and proximal bushing 64 of the impeller is slidable with respect to the shaft.

The axial shaft itself is radially stabilized via a proximal radial bearing 116 and a distal radial bearing 118 (Fig. 5A). In turn, the axial shaft, by passing through lumen 62 defined by the impeller, radially stabilizes the impeller with respect to the inner surface of frame 34, such that even a relatively small gap between the outer edge of the blade of the impeller and the inner surface of frame 34 (e.g., a gap that is as described above) is maintained, during rotation of the impeller.

Referring again to Figs. 3A-B, for some applications, the impeller includes a plurality of elongate elements 67 extending radially from central axial spring 54 to outer helical elongate elements 52. For some applications, as shown, the impeller includes a single integrated impeller-overexpansion-prevention element 72 that defines a plurality of elongate elements 67. For some applications, impeller-overexpansion-prevention element 72 defines a ring 73 and the plurality of elongate elements 67 extending radially from the ring. For some applications, ring 73 of element 72 is placed around (and coupled to) the spring, e.g., by being placed around a tube 70, which is typically disposed at the longitudinally-central location of the spring. The ends of respective elongate elements 67 are then coupled to respective helical elongate elements 52.

For some applications, elongate elements 67 maintain helical elongate element 52 (which defines the outer edge of the impeller blade) within a given distance with respect to the central axial spring. In this manner, the elongate elements are configured to prevent the outer edge of the impeller from being forced radially outward due to forces exerted upon the impeller during the rotation of the impeller. The elongate elements are thereby configured to maintain the gap between the outer edge of the blade of the impeller and the inner surface of frame 34, during rotation of the impeller.

Elongate elements 67 are typically flexible but are substantially non-stretchable along the axis defined by the elongate elements. Typically, each of elongate elements 67 is configured not to resist compression. Rather, each elongate element 67 is configured to exert a tensile force upon helical elongate element 52 that prevents helical elongate element 52 from moving radially outward, such that (in the absence of elongate element 67) a separation between helical elongate element 52 and central axial spring 54 would be greater than a length of elongate element 67. When a force is acting upon the impeller that would cause the helical elongate element 52 to move radially outward (in the absence of elongate element 67), the impeller-overexpansion-prevention element is configured to prevent radial expansion of the impeller. Typically, a respective elongate element 67 is disposed within each one of the impeller blades and is configured to prevent the impeller blade from radially expanding. For some applications, element 72 is made of polyester, and/or another polymer or a natural material that contains fibers, and/or nitinol (or a similar shape-memory alloy).

Typically, impeller 50 is inserted into the left ventricle transcatheterally, while impeller 50 is in a radially-constrained configuration. In the radially-constrained configuration, both helical elongate elements 52 and central axial spring 54 are axially elongated and radially constrained. Typically, film 56 of the material (e.g., silicone and/or a polyurethane) changes shape to conform to the shape changes of the helical elongate elements and the axial support spring, both of which support the film of material. Typically, using a spring to support the inner edge of the film allows the film to change shape without the film becoming broken or collapsing, due to the spring providing a large surface area to which the inner edge of the film bonds. For some applications, using a spring to support the inner edge of the film reduces a diameter to which the impeller can be radially constrained, relative to if, for example, a rigid shaft were to be used to support the inner edge of the film, since the diameter of the spring itself can be reduced by axially elongating the spring.

As described hereinabove, for some applications, proximal bushing 64 of impeller 50 is coupled to axial shaft 92 such that the axial position of the proximal bushing with respect to the shaft is fixed, and distal bushing 58 of the impeller is slidable with respect to the shaft. For example, the proximal bushing may be coupled to coupling element 65 disposed on the axial shaft (shown in Fig. 4), for example via a snap-fit mechanism. For some applications, when the impeller is radially constrained for the purpose of inserting the impeller into the ventricle or for the purpose of withdrawing the impeller from the subject's body, the impeller axially elongates by the distal bushing sliding along the axial shaft distally. Alternatively (not shown), distal bushing 58 of the impeller is coupled to the shaft such that the axial position of the distal bushing with respect to the shaft is fixed, and proximal bushing 64 of the impeller is slidable with respect to the shaft. For some such applications, when the impeller is radially constrained for the purpose of inserting the impeller into the ventricle or for the purpose of withdrawing the impeller from the subject's body, the impeller axially elongates by the proximal bushing sliding along the axial shaft proximally. Subsequent to being released inside the subject's body, the impeller assumes its non-radially-constrained configuration (in which the impeller is typically disposed during operation of the impeller), which is as shown in Figs. 3A-B.

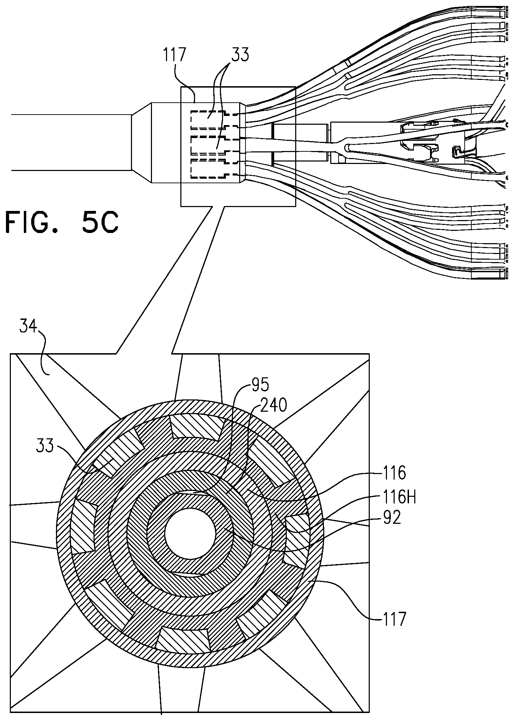

Reference is now made to Figs. 5A and 5B, which are schematic illustrations of impeller 50 and frame 34 of ventricular assist device 20, respectively in non-radially-constrained and radially-constrained states thereof, in accordance with some embodiments. The impeller and the frame are typically disposed in the radially-constrained states during the transcatheteral insertion of the impeller and the frame into the subject's body, and are disposed in the non-radially-constrained states during operation of the impeller inside the subject's left ventricle. Reference is also made to Fig. 5C, which is an enlarged schematic illustration of the proximal end of the frame of the ventricular assist device, in accordance with some embodiments.

As indicated in Fig. 5B, the frame and the impeller are typically maintained in radially-constrained configurations by delivery catheter 143. Typically, in the radially-constrained configuration of the impeller, the impeller has a total length of more than 15 mm (e.g., more than 20 mm), and/or less than 30 mm (e.g., less than 25 mm), e.g., 15-30 mm, or 20-25 mm. Further typically, in the non-radially constrained configuration of the impeller, the impeller has a length of more than 10 mm (e.g., more than 12 mm), and/or less than 20 mm (e.g., less than 18 mm), e.g., 10-20 mm, or 12-18 mm. Typically, when disposed in its non-radially constrained configuration, frame 34 has a total length of more than 25 mm (e.g., more than 30 mm), and/or less than 50 mm (e.g., less than 45 mm), e.g., 25-50 mm, or 30-45 mm. Typically, when disposed in its radially-constrained configuration (within delivery catheter 143), the length of the frame increases by between 2 and 5 mm.

For some applications, when the impeller is disposed in its non-radially-constrained configurations and prior to operation of the impeller, the outer diameter of the impeller at the location at which the outer diameter of the impeller is at its maximum is more than 7 mm (e.g., more than 8 mm), and/or less than 11 mm (e.g., less than 10 mm), e.g., 7-11 mm, or 8-10 mm. For some applications, when frame 34 is disposed in its non-radially-constrained configuration, the inner diameter of frame 34 (as measured from the inside of inner lining 39 on one side of the frame to the inside of inner lining on the opposite side of the frame) is greater than 7.5 mm (e.g., greater than 8.5 mm), and/or less than 10.5 mm (e.g., less than 9.5 mm), e.g., 7.5-10.5 mm, or 8.5-9.5 mm. For some applications, when the frame is disposed in its non-radially-constrained configuration, the outer diameter of frame 34 is greater than 8 mm (e.g., greater than 9 mm), and/or less than 12 mm (e.g., less than 11 mm), e.g., 8-12 mm, or 9-11 mm.

For some applications, when the impeller is disposed in its radially-constrained configuration, e.g., during delivery of the ventricular assist device via delivery catheter 143, the outer diameter of the impeller at the location at which the outer diameter of the impeller is at its maximum is more than 1.5 mm (e.g., more than 2 mm), and/or less than 3 mm (e.g., less than 2.5 mm), e.g., 1.5-3 mm, or 2-2.5 mm. For some applications, the ratio between the outer diameter of the impeller at the location at which the outer diameter of the impeller is at its maximum in (a) the impeller's non-radially constrained configuration versus (b) the impeller's radially constrained configuration is more than 3:1, e.g., more than 7:2, or more than 4:1.

For some applications, when the frame is disposed in its radially-constrained configuration, e.g., during delivery of the ventricular assist device via delivery catheter 143, the outer diameter of the frame is more than 2 mm (e.g., more than 2.5 mm), and/or less than 4 mm (e.g., less than 3.5 mm), e.g., 2-4 mm, or 2.5-3.5 mm. For some applications, the ratio between the outer diameter of the frame in (a) the frame's non-radially constrained configuration versus (b) the frame's radially constrained configuration is more than 5:2, e.g., more than 3:1.

As described hereinabove, typically, axial shaft 92 passes through the axis of impeller 50, via lumen 62 of the impeller. Typically, proximal bushing 64 of the impeller is coupled to the shaft via a coupling element 65 such that the axial position of the proximal bushing with respect to the shaft is fixed, and distal bushing 58 of the impeller is slidable with respect to the shaft. Alternatively, distal bushing 58 of the impeller is coupled to the shaft such that the axial position of the distal bushing with respect to the shaft is fixed, and proximal bushing 64 of the impeller is slidable with respect to the shaft.

The axial shaft itself is radially stabilized via a proximal radial bearing 116 and a distal radial bearing 118. Typically, proximal bearing housing 116H is disposed around, and houses, the proximal bearing, and distal bearing housing 118H is disposed around, and houses, the distal bearing. For some such applications, the radial bearings and the bearing housings are made of respective, different materials from each other. For example, the radial bearings may be made of a first material that has a relatively high hardness, such as ceramic (e.g., zirconia), and the bearing housings may be made of a second material that is moldable into a desired shape, such as a metal or an alloy (e.g., stainless steel, cobalt chromium, and/or nitinol).

For some applications, axial shaft 92 is made of a metal or an alloy, such as stainless steel. For some such applications, the axial shaft is covered with ceramic sleeves 240 (e.g., zirconia sleeves) along regions of the axial shaft that come into contact with either of the proximal and distal bearings 116, 118 during operation of the ventricular assist device. In this manner, the radial interfaces between the axial shaft and the proximal and distal bearings are ceramic-ceramic interfaces. As described in further detail herein, in some embodiments, the impeller and the axial shaft are configured to undergo axial back-and-forth motion during operation of the ventricular assist device. Therefore, for some applications, at locations along the axial shaft corresponding to each of the proximal and distal bearings, the axial shaft is covered with the ceramic sleeve along a length of more than 5 mm, e.g., more than 7 mm. In this manner, over the course of the axial back-and-forth motion of the axial shaft, the ceramic sleeves remain in contact with the radial bearings.

For some applications, along each portion of the axial shaft that is covered with a ceramic sleeve, the shaft is shaped (e.g., via milling, molding, or a different shaping process) to define one or more grooves or indents 95, as shown in the transverse cross-sectional view of Fig. 5C. Alternatively or additionally (not shown), the inner surface of the ceramic sleeve is shaped to define or more grooves or indents. For some such applications, in order to bond the sleeve to the axial shaft, an adhesive is injected into the groove or indent and the adhesive then spreads from the groove or indent across the interface between the axial shaft and the sleeve.

For some applications, the proximal bearing housing 116H and distal bearing housing 118H perform additional functions. Referring first to the proximal bearing housing, as described hereinabove, for some applications, proximal strut junctions 33 of frame 34 are closed around the outside of the proximal bearing housing. For some applications, the outer surface of the proximal bearing housing defines grooves that are shaped such as to receive the proximal strut junctions. For example, as shown, the proximal strut junctions have widened heads, and the outer surface of the proximal bearing housing defines grooves that are shaped to conform with the widened heads of the proximal strut junctions. Typically, securing element 117 (which typically includes a ring) holds the strut junctions in their closed configurations around the outside of proximal bearing housing 116H.

For some applications, additional portions of the ventricular assist device are coupled to the proximal bearing housing. For example, for some applications, drive cable 130 extends from outside the subject's body to axial shaft 92, and is coupled to the axial shaft such that the axial shaft rotates with the drive cable. Typically, the drive cable rotates within a first outer tube 140, which functions as a drive-cable-bearing tube, and which extends from outside the subject's body to the proximal bearing housing. For some applications, the first outer tube is disposed within a second outer tube 142 (also referred to herein as a "delivery tube"), which also extends from outside the subject's body to the proximal bearing housing. For some applications, first outer tube 140 and/or second outer tube 142 is coupled to the proximal bearing housing (e.g., using an adhesive). For example, first outer tube 140 may be coupled to an inner surface of the proximal bearing housing, and second outer tube 142 may be coupled to an outer surface of the proximal bearing housing. Typically, purging fluid is passed between first outer tube 140 and second outer tube 142, e.g., as described with reference to Fig. 15B of WO 24/057252 to Tuval, which is incorporated herein by reference.

Referring now to distal bearing housing 118H, for some applications, distal coupling portion 31 of frame 34 is coupled to an outer surface of distal bearing housing 118H, e.g., via a snap-fit mechanism. For example, the outer surface of a proximal-most portion 119 of the distal bearing housing may include a snap-fit mechanism to which distal coupling portion 31 of frame 34 is coupled. For some applications, distal bearing 118 is disposed within the proximal-most portion 119 of the distal bearing housing, as shown in Fig. 5A. As described hereinabove, for some applications, pump-outlet tube 24 extends to the distal end of frame 34 and defines lateral blood-inlet openings 108. For some such applications, a coupling portion 41 (e.g., a tubular coupling portion) extends distally from the pump-outlet tube, and the coupling portion is coupled to the distal bearing housing in order to anchor the distal end of the pump-outlet tube. For some applications, an intermediate portion 123 of the distal bearing housing defines a ridged or a threaded outer surface, to which coupling portion 41 of the pump-outlet tube is coupled (e.g., via an adhesive). For some applications, the outer surface is ridged in order to enhance bonding between the distal bearing housing and coupling portion 41 of the pump-outlet tube. For some applications, the outer surface is threaded in order to enhance bonding between the distal bearing housing and coupling portion 41 of the pump-outlet tube and to facilitate the application of adhesive between the outer surface and coupling portion 41 of the pump-outlet tube. For some applications, a distal portion 121 of the distal bearing housing is configured to stiffen a region of distal-tip element 107 into which the distal end of shaft 92 moves (e.g., axial-shaft-receiving tube 126, or a portion thereof). Typically, distal-tip element 107 is coupled to an outer surface of distal portion 121 of the distal bearing housing (e.g., via adhesive). For some applications, at least a portion of the outer surface of distal portion 121 of the distal bearing housing is ridged and/or threaded in order to enhance bonding between distal-tip element 107 and the distal bearing housing.

As described above, axial shaft 92 is radially stabilized via proximal radial bearing 116 and distal radial bearing 118. In turn, the axial shaft, by passing through lumen 62 defined by the impeller, radially stabilizes the impeller with respect to the inner surface of frame 34 and inner lining 39, such that even a relatively small gap between the outer edge of the blade of the impeller and inner lining 39 (e.g., a gap that is as described above) is maintained, during rotation of the impeller, as described hereinabove. Typically, the impeller itself is not directly disposed within any radial bearings or thrust bearings. Rather, bearings 116 and 118 act as radial bearings with respect to the axial shaft.

In some embodiments, pump-head portion 27 (and more generally ventricular assist device 20) does not include any thrust bearing that is configured to be disposed within the subject's body and that is configured to oppose thrust generated by the rotation of the impeller. For some applications, one or more thrust bearings are disposed outside the subject's body (e.g., within motor unit 23, shown in Fig. 1A), and opposition to thrust generated by the rotation of the impeller is provided solely by the one or more thrust bearings disposed outside the subject's body. For some applications, a mechanical element and/or a magnetic element is configured to maintain the impeller within a given range of axial positions. For example, a magnet that is disposed at the proximal end of the drive cable (e.g., outside the subject's body) may be configured to impart axial motion to the impeller, and/or to maintain the impeller within a given range of axial positions.

In alternate embodiments, axial shaft 92 is omitted, and the impeller is instead coupled to a distal portion of drive cable 130; for example, the drive cable may pass through lumen 62 of the impeller. In other words, the distal portion of the drive cable may function as an axial shaft. It should thus be understood that throughout the present description, the distal portion of the drive cable, which may also be referred to as an "axial shaft," may substitute for axial shaft 92.

Typically, the space between delivery catheter 143 and delivery tube 142 functions as an aortic-pressure sensing channel. During operation of the left ventricular assist device, the distal end of the delivery catheter is typically disposed in the subject's descending aorta, such that this channel is exposed to the aortic bloodstream and aortic blood pressure.

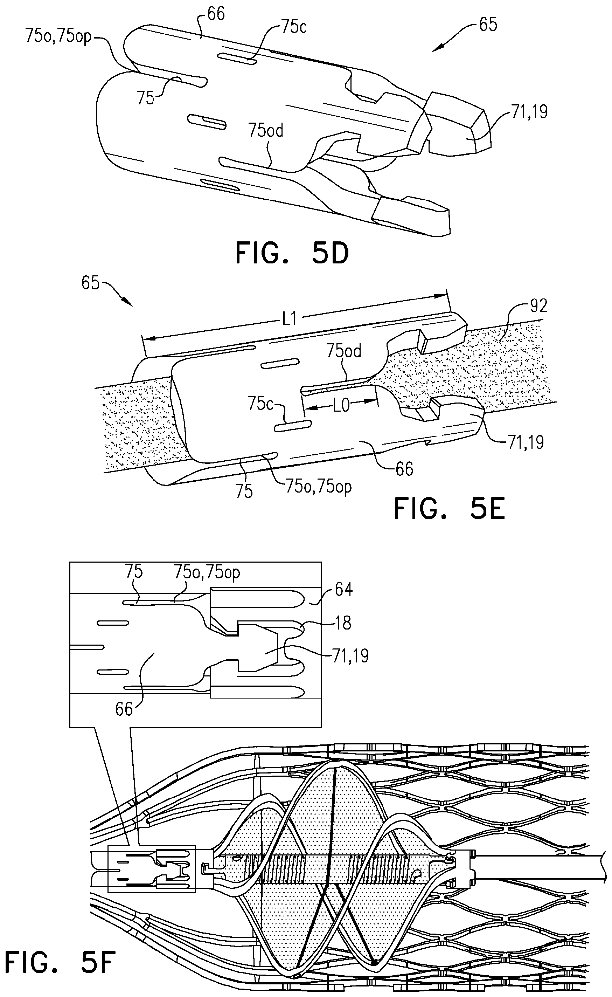

Reference is now made to Fig. 5D, 5E, and 5F, which are schematic illustrations of coupling element 65, in accordance with some embodiments. As described hereinabove, for some applications, proximal bushing 64 of impeller 50 is coupled to axial shaft 92 such that the axial position of the proximal bushing with respect to the shaft is fixed, and distal bushing 58 of the impeller is slidable with respect to the shaft. For some applications, the proximal bushing is coupled to the axial shaft via coupling element 65, for example via a snap-fit mechanism. Typically, the coupling element includes a first region (or "portion") 66 disposed around axial shaft 92, and a second region (or "portion") 71, which may also be disposed around the axial shaft.

The coupling element is coupled to proximal bushing 64 at second region 71. This coupling may be effected via a snap-fit mechanism, as noted above. For example, second region 71 may be shaped to define one or more protrusions 19, proximal bushing 64 may be shaped to define one or more indentations 18, and the proximal bushing may couple to second region 71 by virtue of protrusions 19 snapping into indentations 18. Alternatively, the proximal bushing may be shaped to define protrusions 19, second region 71 may be shaped to define indentations 18, and the proximal bushing may couple to second region 71 by virtue of the protrusions snapping into the indentations.

The coupling element is coupled to axial shaft 92 at first region 66. For example, for some applications, the first region of the coupling element is welded to the shaft. For other applications, the coupling element (or at least first region 66) is made of a shape-memory material (e.g., a shape-memory alloy, such as nitinol or cobalt chromium). For example, the coupling element may comprise a tube of the shape-memory material that is cut to define the first and second regions. For some such applications, at least the first region of the coupling element (or the entire coupling element) is shape set to have an inner diameter that is smaller (e.g., between 0.01 and 0.1 mm smaller) than the outer diameter of the axial shaft. For example, the axial shaft may have an outer diameter of 0.9 mm and the inner diameter of the first region of the coupling element may be between 0.85 and 0.89 mm (e.g., 0.87 mm). Thus, following the placement of the first region around the axial shaft, the first region becomes radially contracted around, and thus locked in place with respect to, the axial shaft. For some applications, coupling the coupling element to the axial shaft via this method, rather than via welding, is desirable, since the coupling element and/or the axial shaft can be weakened by being heated during the welding.

For some applications, the first region of the coupling element is shaped to define one or more slits 75, e.g., by virtue of comprising a tube that defines slits 75. Slits 75 facilitate a radial expansion of the first region such that the first region is placeable around the axial shaft. Following the placement around the axial shaft, the first region may radially contract around the axial shaft, as described above.

Slits 75 may incorporate various features for facilitating the expansion of first region 66. For example, in some embodiments, one or more of slits 75 are open-ended slits 75o, each of which has an open end. Open-ended slits 75o may include one or more proximally-open slits 75op, which are open at the proximal end of first region 66, and/or one or more distally-open slits 75od, which are open at the distal end of the first region. Optionally, the length L0 of each of the open-ended slits may be 5-40 percent of the length L1 of the coupling element. Alternatively or additionally to open-ended slits 75o, one or more of slits 75 may be closed-ended slits 75c, each of which does not have any open end. In some embodiments, as shown in Figs. 5D-F, closed-ended slits 75c alternate with open-ended slits 75o around the circumference of first region 66.

During manufacture of the blood pump, first region 66 is placed around axial shaft 92 such that, as described above, the first region becomes radially contracted around the axial shaft. Typically, in addition to first region 66, second region 71 is placed around the axial shaft.

Subsequently to coupling the coupling element to the axial shaft, the impeller is coupled to the axial shaft, by coupling proximal bushing 64 to the second region of the coupling element. As described above, this coupling may be performed via a snap-fit mechanism; for example, protrusions 19 may be snapped into indentations 18. Thus, as the axial shaft rotates, the blades of the impeller rotate, thereby pumping blood of the subject.

In alternate embodiments, second region 71 is coupled to distal bushing 58 (e.g., via a snap-fit mechanism, as described), such that the distal bushing is fixed in place with respect to the axial shaft, and proximal bushing 64 is slidable along the axial shaft.

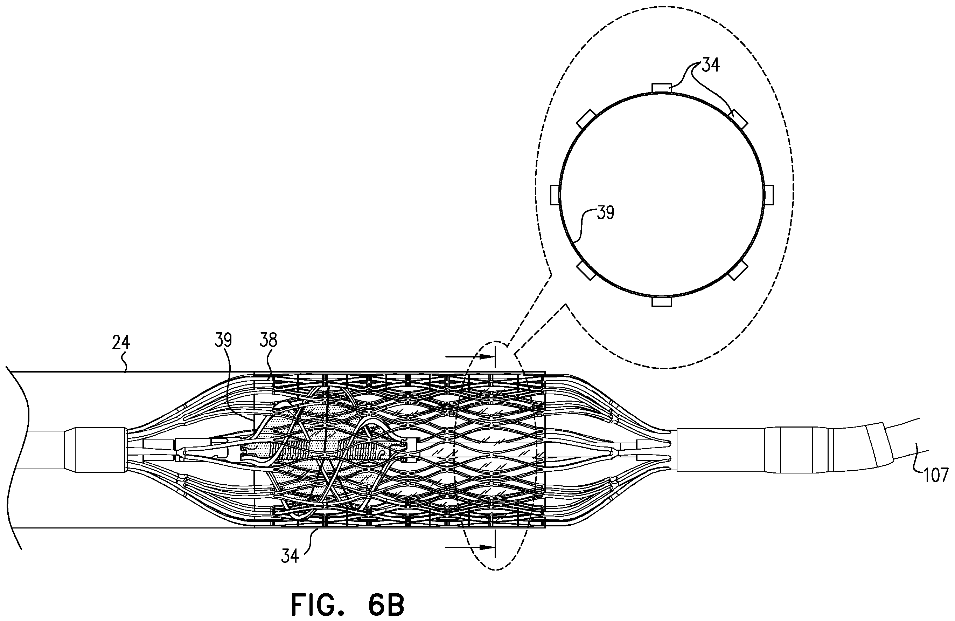

Reference is now made to Figs. 6A and 6B, which are schematic illustrations of ventricular assist device 20, the device including inner lining 39 that lines the inside of frame 34 that houses impeller 50, in accordance with some embodiments.

For some applications, inner lining 39 lines the inside of frame 34 (e.g., by virtue of being bonded to the frame), in order to provide a smooth inner surface (e.g., a smooth inner surface having a substantially circular cross-sectional shape) through which blood is pumped by impeller. Typically, by providing a smooth surface, the covering material reduces hemolysis that is caused by the pumping of blood by the impeller, relative to if the blood were pumped between the impeller and struts of frame 34. For some applications, inner lining 39 includes a polyurethane, polyester, and/or silicone. Alternatively or additionally, the inner lining includes polyethylene terephthalate (PET) and/or polyether block amide (e.g., PEBAX^{®}).

Typically, the inner lining is disposed over the inner surface of at least a portion of central cylindrical portion 38 of frame 34. For some applications, pump-outlet tube 24 also covers central cylindrical portion 38 of frame 34 around the outside of the frame, for example, such that pump-outlet tube 24 and inner lining 39 overlap over at least 50 percent of the length of the inner lining, for example, over the entire length of the cylindrical portion of frame 34, e.g., as shown in Fig. 6A. For some applications, there is only partial overlap between pump-outlet tube 24 and inner lining 39, e.g., as shown in Fig. 6B. For example, pump-outlet tube 24 may overlap with inner lining 39 along less than 50 percent (e.g., along less than 25 percent) of the length of the inner lining. For some such applications, during insertion of ventricular assist device 20 into the subject's body, the impeller is advanced distally within frame 34, such that the impeller is not disposed within the area of overlap between the pump-outlet tube and the inner lining, such that there is no longitudinal location at which the impeller, pump-outlet tube 24, frame 34, and inner lining 39 all overlap with each other. As shown in Figs. 6A and 6B, for some applications, a single axially-facing blood-inlet opening 108 is defined at the distal end of the pump-outlet tube and/or the inner lining. Alternatively, the inner lining is disposed over the inner surface of at least a portion of central cylindrical portion 38 of frame 34, and the pump-outlet tube extends to the distal end of the frame and defines a plurality of lateral blood-inlet openings 108. Such applications are described in further detail hereinbelow with reference to Figs. 7A-D, for example.

Typically, over the area of overlap between inner lining 39 and pump-outlet tube 24, the inner lining is shaped to form a smooth surface (e.g., in order to reduce hemolysis, as described hereinabove), and pump-outlet tube 24 is shaped to conform with the struts of frame 34 (e.g., as shown in the cross-section in Fig. 6A). Further typically, the inner lining has a substantially circular cross-section (for example, due to the relatively small cell width within the central cylindrical portion of the frame, as described hereinabove with reference to Fig. 2). For some applications, over the area of overlap between inner lining 39 and pump-outlet tube 24, the pump-outlet tube and the inner lining are coupled to each other, e.g., via vacuum, via an adhesive, and/or using a heat-welding procedure, which typically includes thermoforming of the pump-outlet tube, as described below.

For some applications, inner lining 39 and pump-outlet tube 24 are made of different materials (e.g., different classes of polymer) from each other. For example, the inner lining may be made of a polyurethane, and the pump-outlet tube may be made of polyether block amide (e.g., PEBAX^{®}). Alternatively, for example, the inner lining and the pump-outlet tube may be made of different types of the same class of polymer, e.g., different types of polyurethane. Alternatively, inner lining 39 and pump-outlet tube 24 are made of the same material as each other. For example, both the inner lining and the pump-outlet tube may be made of the same type of polyurethane (i.e., the same polyurethane polymer), or of polyether block amide (e.g., PEBAX^{®}).

For some embodiments, regardless of whether the inner lining and pump-outlet tube are made of the same material (e.g., the same polyurethane polymer) or of different materials (e.g., different polyurethane polymers or different classes of polymer), the inner lining has a flexural modulus of more than 0.1 GPa and/or less than 2 GPa, e.g., between 0.1 GPa and 2 GPa, and the pump-outlet tube has a flexural modulus of more than 0.1 GPa and/or less than 0.8 GPa (e.g., less than 0.5 GPa), e.g., between 0.1 GPa and 0.8 GPa, or between 0.1 GPa and 0.5 GPa.

Typically, the pump-outlet tube has a flexural modulus of more than 0.1 GPa and/or less than 0.8 GPa (e.g., less than 0.5 GPa), e.g., between 0.1 GPa and 0.8 GPa, or between 0.1 GPa and 0.5 GPa. Thus, the pump-outlet tube is typically configured such as to be sufficiently flexible that that edges defined by the pump-outlet tube (e.g., the edges of blood-outlet openings 109) do not injure tissue of the subject (e.g., the aortic wall).

As described above, typically the inner lining has a flexural modulus of more than 0.1 GPa and/or less than 2 GPa, e.g., between 0.1 GPa and 2 GPa. For some applications, the inner lining has a flexural modulus of more than 0.8 GPa (e.g., more than 1 GPa) and/or less than 2 GPa (e.g., less than 1.5 GPa), e.g., between 0.8 GPa and 2 GPa, or between 1 GPa and 1.5 GPa. For some applications, by having a flexural modulus of more than 0.8 GPa (e.g., more than 1 GPa), the inner lining is configured to support frame 34 in an open configuration, when the ventricular assist device is in a deployed state. For some applications, by having a flexural modulus of more than 0.8 GPa (e.g., more than 1 GPa), the inner lining is able to perform its function of lining the frame (and, optionally, supporting the frame in an open configuration), while having a relatively small thickness, e.g., a thickness less than 0.3 mm, such that the pump-head portion of the device can be more easily contained in its radially-constrained configuration. Alternatively, the inner lining has a flexural modulus of more than 0.1 GPa and/or less than 0.8 GPa (e.g., less than 0.5 GPa), e.g., between 0.1 GPa and 0.8 GPa, or between 0.1 GPa and 0.5 GPa.

For some applications, the inner lining and the pump-outlet tube are made from the same material as one another (e.g., a type of polyurethane) and both the inner lining and the pump-outlet tube have a flexural modulus of more than 0.1 GPa and/or less than 0.8 GPa (e.g., less than 0.5 GPa), e.g., between 0.1 GPa and 0.8 GPa, or between 0.1 GPa and 0.5 GPa. Alternatively, the inner lining and the pump-outlet tube are made from different types of the same class of polymer (e.g., different types of polyurethane). For some such applications, (a) the inner lining has a flexural modulus of more than 0.8 GPa (e.g., more than 1 GPa) and/or less than 2 GPa (e.g., less than 1.5 GPa), e.g., between 0.8 GPa and 2 GPa, or between 1 GPa and 1.5 GP, and (b) the pump-outlet tube has a flexural modulus of more than 0.1 GPa and/or less than 0.8 GPa (e.g., less than 0.5 GPa), e.g., between 0.1 GPa and 0.8 GPa, or between 0.1 GPa and 0.5 GPa.

Typically, the respective melting temperatures of the inner lining and the pump-outlet tube are within 20 °C of one another, e.g., within 10 °C, or within 5 °C of one another. In particular, in embodiments in which the inner lining and the pump-outlet tube are made of the same class of polymer (e.g., different types of the same class of polymer or the same type of polymer), the respective melting temperatures of the inner lining and the pump-outlet tube are within 20 °C of one another, e.g., within 10 °C, or within 5 °C of one another. The similar melting temperatures facilitate heat welding the inner lining to the pump-outlet tube, as described below.

Further typically, the respective glass-transition temperatures of the inner lining and the pump-outlet tube are within 20 °C of one another, e.g., within 10 °C, or within 5 °C of one another. In particular, in embodiments in which the inner lining and the pump-outlet tube are made of the same class of polymer (e.g., different types of the same class of polymer or the same type of polymer), the respective glass-transition temperatures of the inner lining and the pump-outlet tube are within 20 °C of one another, e.g., within 10 °C, or within 5 °C of one another. The similar melting temperatures facilitate heat welding the inner lining to the pump-outlet tube, as described below.

Typically, the pump-outlet tube is fixed over cylindrical portion 38 of frame 34 by virtue of being bonded or otherwise coupled to the cylindrical portion of the frame and/or to inner lining 39.

For some applications, the inner lining is directly bonded to the inner surface of the frame before the pump-outlet tube is bonded to the outside of the frame. It is noted that, by bonding the inner lining directly to the inner surface of the frame (rather than simply bonding the inner lining to the pump-outlet tube and thereby sandwiching the frame between the inner lining to the pump-outlet tube), any air bubbles, folds, and other discontinuities in the smoothness of the surface provided by the inner lining are typically avoided.

For example, in some embodiments, the inner lining, which is shaped as a tube, is placed over a mandrel having the desired diameter of the inner lining. The mandrel is then heated to a temperature that is higher than the glass-transition temperature of the inner lining but below the melting temperature of the inner lining. The heat causes the inner lining to shrink around the mandrel, such that the desired diameter of the inner lining is obtained. Subsequently, the frame (typically, the central cylindrical portion of the frame) is placed over the inner lining, and pressure is applied to the frame while the assembly of the mandrel, inner lining, and frame is heated in an oven. The heat and pressure cause the frame to bond to the inner lining.

For some applications, similar techniques to those described hereinabove for enhancing bonding between the elastomeric film and the helical elongate elements of the impeller, are used to enhance bonding between the inner lining and the inner surface of the frame. For example, in some applications, initially, the frame is treated so as to enhance bonding between the inner lining and the inner surface of the frame. For some applications, the treatment of the frame includes applying a plasma treatment to the frame (e.g., to the inner surface of the frame), dipping the frame in a coupling agent that has at least two functional groups that are configured to bond respectively with the frame and with the material from which the inner lining is made (e.g., a silane solution), dipping the frame in a solution that contains the material from which the inner lining is made (e.g., a polyurethane solution), and/or spraying such a solution over the inner surface of the frame. For some applications, the inner lining is made of an elastomeric material (e.g., a polyurethane) and the coupling agent is a silane solution, such as a solution of n-(2-aminoethyl)-3-aminopropyltrimethoxysilane, with the silane containing a first functional group (e.g., (OH)) which is configured to bond with the frame (which is typically made of an alloy, such a nitinol), and the silane containing a second functional group (e.g., (NH2)) which is configured to bond with the elastomeric material.

Subsequently to the inner lining having been bonded to the frame, a portion of pump-outlet tube 24 is placed around the outside of the frame, and heat and pressure are applied. Typically, at this stage, the assembly is heated to a heat-welding temperature that is above the glass-transition temperatures of at least one of the pump-outlet tube 24 and inner lining 39, such that pump-outlet tube 24 is heat welded to the inner lining. Typically, a heat-welding temperature is selected such that it is high enough to cause at least one of the inner lining and the pump-outlet tube to soften so as to become welded to the other portion, yet is not so high so as to melt either one of these components.

As noted above, in some embodiments in which the inner lining and the pump-outlet tube are made of the same class of polymer (e.g., different types of the same class of polymer or the same type of polymer), the respective melting temperatures of the inner lining and the pump-outlet tube are within 20 °C of one another, e.g., within 10 °C, or within 5 °C of one another. In some embodiments in which the inner lining and the pump-outlet tube are made of the same class of polymer (e.g., different types of the same class of polymer or the same type of polymer), the respective glass-transition temperatures of the inner lining and the pump-outlet tube are within 20 °C of one another, e.g., within 10 °C, or within 5 °C of one another. Advantageously, the similar melting temperatures and/or glass-transition temperatures facilitate selecting a heat-welding temperature that is high enough to cause both the inner lining and the pump-outlet tube to soften so as to become welded to one another, yet is not so high so as to melt either one of these components.

In some embodiments, during the heat-welding process, the frame is heated from inside the frame, using the mandrel. Typically, while the frame is heated, an outer tube (which is typically made from silicone) applies pressure to pump-outlet tube 24 that causes pump-outlet tube 24 to be pushed radially inwardly, in order to cause the pump-outlet tube to conform with the shapes of the struts of the frame, as shown in the cross-section of Fig. 6A. For some applications, the mandrel is shorter than the inner lining, such that margins are left outside of the mandrel at each of the ends of the inner lining. Thus, the inner lining acts as a shield to protect the pump-outlet tube from being overheated and damaged. In other words, the margins prevent the mandrel from coming into direct contact with the frame and/or the pump-outlet tube. In other embodiments, the heat-welding process is performed in an oven.

For some applications, following the heat welding, the combination of the frame, the inner lining, and the portion of pump-outlet tube 24 disposed around the frame is shape set to a desired shape and desired dimensions using shape setting techniques that are known in the art.

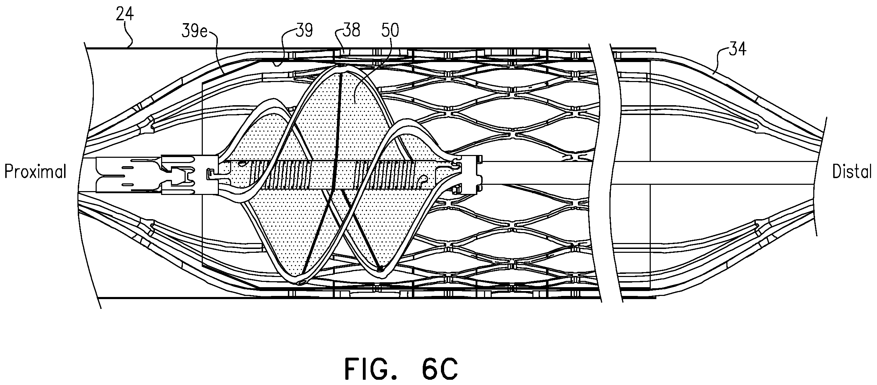

Reference is now made to Fig. 6C, which is a schematic illustration of an inner lining that includes an extension 39e extending proximally beyond cylindrical portion 38 of frame 34, in accordance with some embodiments. For some applications, the inner lining extension 39e is not coupled to the inner surface of frame 34, but rather, the material that comprises the extension is free to flap within the blood flow that is generated by the impeller. In some cases, the inner lining extension increases the efficiency of pumping of the blood by the impeller, for example by rectifying non-linear flow paths of the blood that are generated by the pumping of the impeller.

Reference is now made to Figs. 7A-D, which are schematic illustrations of pump-outlet tube 24 or a portion thereof, the pump-outlet tube being configured to define lateral blood-inlet openings 108 at a distal end thereof, in accordance with some embodiments. For some applications, the pump-outlet tube extends substantially until the distal end of distal conical portion 40 of frame 34. For such applications, the pump-outlet tube typically defines a distal conical portion 46 which is distally facing, i.e., facing such that the narrow end of the cone is distal with respect to the wide end of the cone. Typically, the pump-outlet tube includes coupling portion 41 (e.g., a tubular coupling portion, as shown), which extends distally from the pump-outlet tube. As described hereinabove, the coupling portion is coupled to the distal bearing housing in order to anchor the distal end of the pump-outlet tube.

For some applications (not shown), the pump-outlet tube defines two to four lateral blood-inlet openings. Typically, for such applications, each of the blood-inlet openings defines an area of more than 20 square mm (e.g., more than 30 square mm), and/or less than 60 square mm (e.g., less than 50 square mm), e.g., 20-60 square mm, or 30-50 square mm. Alternatively or additionally, the outlet tube defines a greater number of smaller blood-inlet openings 108, e.g., more than 10 blood-inlet openings, more than 10 blood-inlet openings, more than 100 blood-inlet openings, more than 200 blood-inlet openings, or more than 300 blood-inlet openings, e.g., 50-100 blood-inlet openings, 100-300 blood-inlet openings, or 300-500 blood-inlet openings.. For some applications, the blood-inlet openings are sized such as (a) to allow blood to flow from the subject's left ventricle into the tube and (b) to block structures from the subject's left ventricle from entering into the frame. Typically, for such applications, the distal conical portion 46 of pump-outlet tube 24 is configured to reduce a risk of structures from the left ventricle (such as chordae tendineae, trabeculae carneae, and/or papillary muscles) entering into frame 34 and potentially being damaged by the impeller and/or the axial shaft, and/or causing damage to the left ventricular assist device. Therefore, for some applications, the blood-inlet openings are shaped such that, in at least one direction, the widths (or spans) of the openings are less than 1 mm, e.g., 0.1-1 mm, or 0.2-0.6 mm. By defining such a small width (or span), it is typically the case that structures from the left ventricle (such as chordae tendineae, trabeculae carneae, and/or papillary muscles) are blocked from entering into frame 34. For some such applications, each of the blood-inlet openings defines an area of more than 0.05 square mm (e.g., more than 0.1 square mm), and/or less than 3 square mm (e.g., less than 1 square mm), e.g., 0.05-3 square mm, or 0.1-1 square mm. Alternatively, each of the blood-inlet openings defines an area of more than 0.1 square mm (e.g., more than 0.3 square mm), and/or less than 5 square mm (e.g., less than 1 square mm), e.g., 0.1-5 square mm, or 0.3-1 square mm.

Typically, the portion of the pump-outlet tube that defines the blood-inlet openings has a porosity of more than 40 percent, e.g., more than 50 percent, more than 60 percent, or more than 70 percent (where porosity is defined as the percentage of the area of this portion that is porous to blood flow). Thus, on the one hand, the blood-inlet openings are relatively small (in order to prevent structures of the left ventricular from entering the frame), but on the other hand, the porosity of the portion of the pump-outlet tube that defines the blood-inlet openings is relatively high, such as to allow sufficient blood flow into the pump-outlet tube.

For some applications, each of the blood-inlet openings has a circular or a polygonal shape. For some applications, each of the blood-inlet openings has a hexagonal shape, as shown in Figs. 7A-D. Typically, using openings having a hexagonal shape allows the portion of the pump-outlet tube that defines the blood-inlet openings to have a relatively high porosity (e.g., as described hereinabove), while providing the portion of the pump-outlet tube that defines the blood-inlet openings with sufficient material between the blood-inlet openings to prevent tearing and/or stretching of the material. As shown in Fig. 7B, for some applications, a width W of gaps between adjacent hexagonal (or other polygonal) holes is more than 0.01 mm (e.g., more than 0.02 mm), and/or less than 0.2 mm (e.g., less than 0.15 mm), for example, 0.01-0.2 mm, or 0.02-0.15 mm. For some applications, the distance D between opposing sides of each of the hexagons (or other types of polygons) is more than 0.1 mm (e.g., more than 0.2 mm) and/or less than 0.8 mm (e.g., less than 0.6 mm), e.g., 0.1-0.8 mm, or 0.2-0.6 mm. As indicated in Fig. 7B, typically each of the polygons encloses a circle (such that any structure that cannot pass through such a circle would be unable to pass through the polygon). Typically, the diameter of the circle enclosed by the polygon is the equivalent of distance D, e.g., more than 0.1 mm (e.g., more than 0.2 mm) and/or less than 0.8 mm (e.g., less than 0.6 mm), e.g., 0.1-0.8 mm, or 0.2-0.6 mm.

The scope of the present disclosure includes having uniformly sized and/or shaped lateral blood-inlet openings (e.g., circular, rectangular, polygonal, and/or hexagonal lateral blood-inlet openings). Similarly, the scope of the present disclosure includes a distal conical portion 46 of the pump-outlet tube that defines lateral blood-inlet openings being arranged such that the distal conical portion has a uniform porosity, with the porosity being substantially uniform over different regions of the distal conical portion.

The scope of the present disclosure further includes having non-uniformly sized and/or shaped lateral blood-inlet openings (e.g., circular, rectangular, polygonal, and/or hexagonal lateral blood-inlet openings), disposed in any arrangement along the distal conical portion 46 of the pump-outlet tube. Similarly, the scope of the present disclosure includes a distal conical portion 46 of the pump-outlet tube that defines lateral blood-inlet openings being arranged such that the distal conical portion has a non-uniform porosity, with the porosity varying over different regions of the distal conical portion. For some applications, the shapes and/or sizes of the lateral blood-inlet openings, and/or the porosity of the distal conical portion, is varied such as to account for varying blood flow dynamics at different regions of the distal conical portion. Alternatively or additionally, the shapes and/or sizes of the lateral blood-inlet openings, and/or the porosity of the distal conical portion, is varied such as to account for changes in the shape of the distal conical portion along its length.

For some applications, along distal conical portion 46 of pump-outlet tube 24, the thickness of the polymeric material from which the pump-outlet tube is made is greater than the thickness in other regions of the pump-outlet tube (e.g., within the central cylindrical portion and/or the proximal conical portion of the pump-outlet tube). For some such applications, pump-outlet tube 24 is manufactured in this manner in order to prevent tearing of the tube within the distal conical portion 46, which defines blood-inlet openings 108, and may (in some cases) be at greater risk of tearing than other portions of the pump-outlet tube.

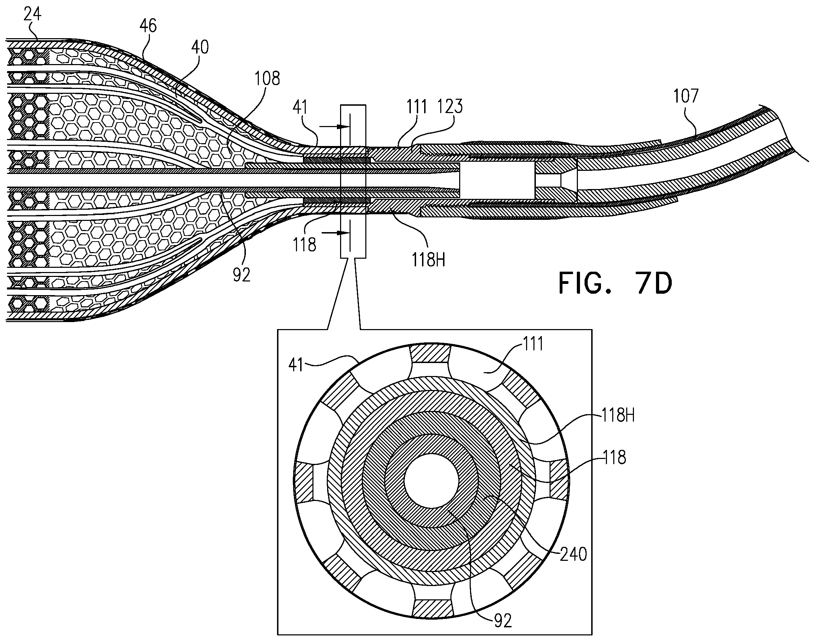

Reference is now made to Fig. 7D, which is an enlarged schematic illustration of the interface between the distal end of pump-outlet tube 24 and distal-tip element 107. Typically, the pump-outlet tube includes a coupling portion 41 (e.g., a tubular coupling portion, as shown), which extends distally from the pump-outlet tube. As described hereinabove, the coupling portion is coupled to distal bearing housing 118H in order to anchor the distal end of the pump-outlet tube. Also as described hereinabove, typically, the pump-outlet tube is coupled to the outside of the central cylindrical portion of the frame. For some applications, distal conical portion 46 of the pump-outlet tube is not itself bonded to distal conical portion 40 of the frame. Rather, distal conical portion 46 of the pump-outlet tube is held in place with respect to distal conical portion 40 of the frame, by virtue of coupling portion 41 being coupled to distal bearing housing 118H and the pump-outlet tube being coupled to the outside of the central cylindrical portion of the frame. Alternatively, the distal conical portion 46 of the pump-outlet tube is directly coupled to distal conical portion 40 of the frame (e.g., via heat shrinking).

As described hereinabove, for some applications, coupling portion 41 is coupled to the outer surface of portion 123 of distal bearing housing 118H. For some applications, coupling portion 41 defines a hole 111 (e.g., toward the distal end of the coupling portion), as shown in Fig. 7D. For some applications, an adhesive is applied between coupling portion 41 and the outer surface of portion 123 of distal bearing housing 118H, via the hole. For some applications, the outer surface of portion 123 of distal bearing housing 118H is threaded. Typically, the threaded outer surface allows the adhesive to gradually and uniformly spread between coupling portion 41 and the outer surface of portion 123 of distal bearing housing 118H. Further typically, the coupling portion is transparent, such that the spread of the adhesive is visible through the coupling portion. Therefore, for some applications, once the adhesive has sufficiently spread between coupling portion 41 and the outer surface of portion 123 of distal bearing housing 118H (e.g., once the outer surface of portion 123 has been covered with the adhesive), application of the adhesive is terminated.

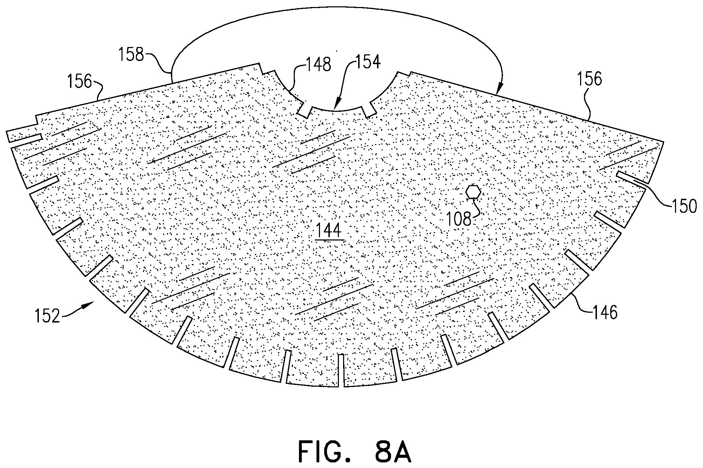

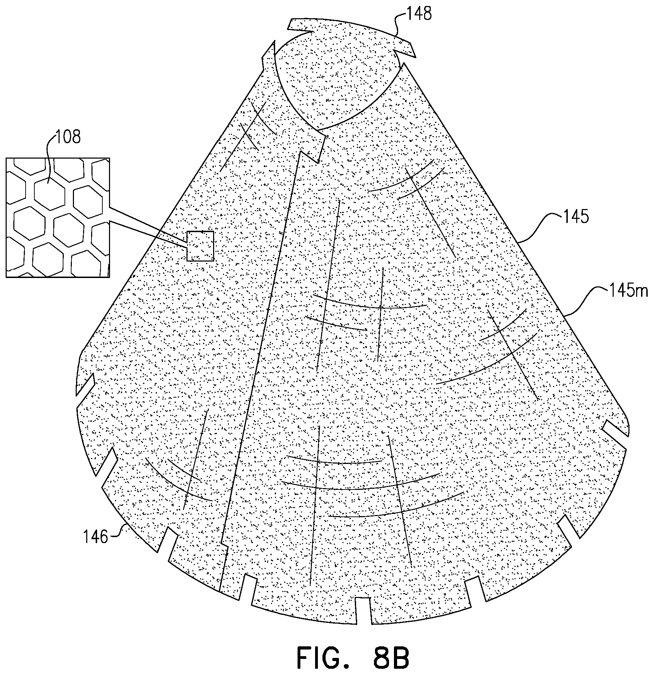

Reference is now made to Fig. 8A, which is a schematic illustration of a sheet 144 of material, such as a polyether block amide, a polyether ether ketone, or another polymer, and to Fig. 8B, which is a schematic illustration of a frustoconical inlet guard 145 formed from sheet 144, in accordance with some embodiments.

In some embodiments, sheet 144 is provided for forming inlet guard 145, which provides at least some of the function of distal portion 46 (Fig. 7D) of the pump-outlet tube. Blood-inlet openings 108, which may have any of the properties described above with reference to distal portion 46 of the pump-outlet tube, are formed in sheet 144, e.g., via laser cutting. Subsequently, sheet 144 is rolled so as to form the frustoconical inlet guard. Thus, this method of forming the inlet guard takes advantage of the fact that it is easier to form holes in a flat piece of material, rather than a three-dimensional structure.

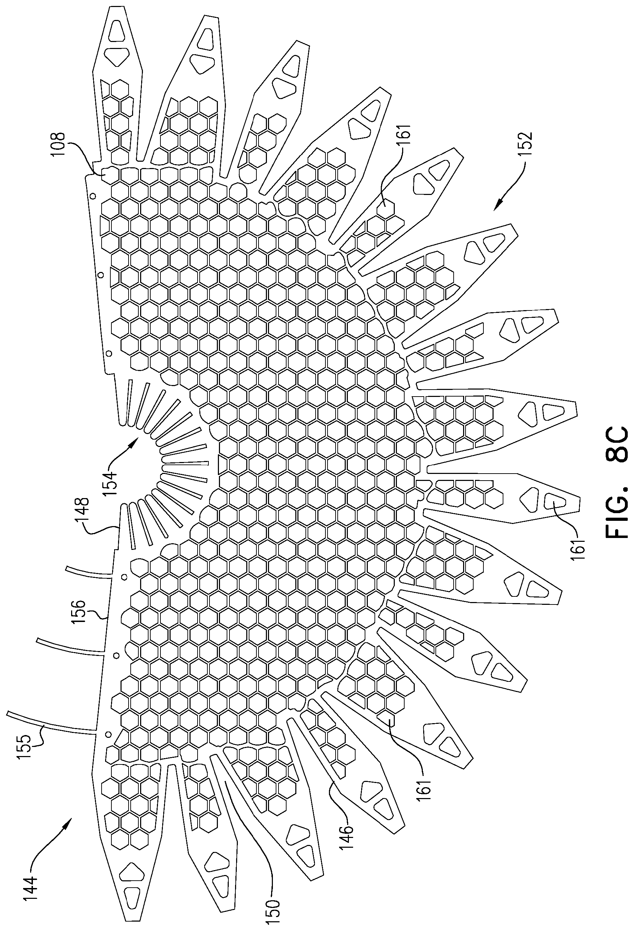

As shown in Fig. 8A, the shape of sheet 144 is generally that of a section of a torus, comprising a longer arced proximal end 152, a shorter arced distal end 154, and opposing lateral edges 156. To roll the sheet, one lateral edge 156 is coupled (e.g., bonded) to the surface of the sheet near the opposite lateral edge, as indicated by a rolling indicator 158. One or more tabs extending from the sheet may facilitate this rolling, as further described below with reference to Fig. 8C. Following the formation of the inlet guard, the inlet guard is coupled to the pump-outlet tube, e.g., to the distal end of cylindrical portion 44 (Fig. 1C).

In some embodiments, multiple proximal flaps 146 are formed in the sheet of material, e.g., by cutting slits 150 in the proximal end of the sheet so as to define flaps 146. The inlet guard thus comprises a frustoconical main body 145m, which is shaped to define the blood-inlet openings, and proximal flaps 146. The inlet guard is coupled to the pump-outlet tube by coupling proximal flaps 146 to the pump-outlet tube. Advantageously, flaps 146 help prevent folding or creasing in the inlet guard.

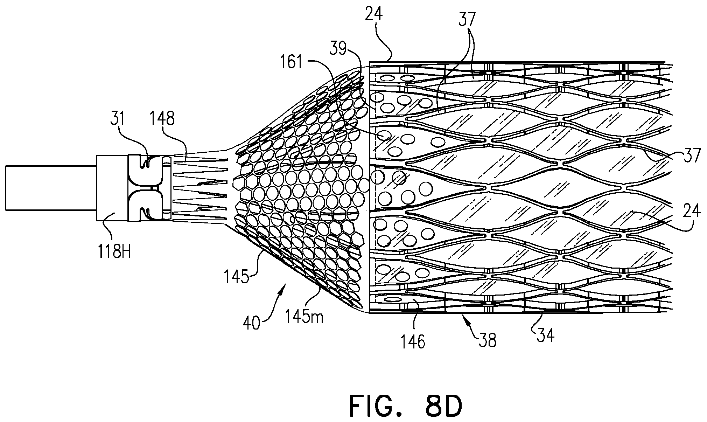

In some embodiments, the inlet guard is also coupled to inner lining 39 (Fig. 7A). For example, as shown in Fig. 8D, which is described below, proximal flaps 146 may be coupled to the inner lining, such that the proximal flaps are sandwiched between the inner lining and the pump-outlet tube.

As a specific example, for some applications, the inner lining and the pump-outlet tube are heat welded to one another, e.g., as described above with reference to Figs. 6A-B, while the proximal flaps are between the inner lining and the pump-outlet tube. In some such embodiments, to protect the inlet guard from degradation during the heat-welding process, the heat-welding temperature (to which the inner lining and the pump-outlet tube are heated) is lower than the glass-transition temperature of the inlet guard but higher than the glass-transition temperature of at least one of the inner lining and the pump-outlet tube (and in some embodiments, higher than the glass-transition temperature of both of the inner lining and the pump-outlet tube). Furthermore, as described above, the heat-welding temperature is typically lower than the respective melting points of the inner lining and the pump-outlet tube, such that the pump-outlet tube is bonded to the inner lining without deformation of the inner lining or pump-outlet tube.

In some embodiments, the inlet guard is made of the same material as the inner lining and/or the pump-outlet tube, such as a polyurethane (e.g., Pellethane^{®}) or a polyether ether ketone. In other embodiments, the inlet guard is made of a different material. In some such embodiments, the glass-transition temperature of the material of which the inlet guard is made is higher than the respective glass-transition temperatures of each of the inner lining and the pump-outlet tube. The heat-welding temperature is lower than the glass-transition temperature of the material but higher than the respective glass-transition temperatures of each of the inner lining and the pump-outlet tube.

For example, in some applications, the inner lining is made of a polyurethane (e.g., Pellethane^{®}), the pump-outlet tube is made of polyether block amide (e.g., PEBAX^{®}), and the inlet guard is made of a polyether ether ketone. The heat-welding temperature is lower than the glass-transition temperature of the polyether ether ketone but higher than the respective glass-transition temperatures of each of the polyurethane and polyether block amide. Alternatively, the inner lining and pump-outlet tube are made of the same type or different types of polyurethane, and the inlet guard is made of a polyether ether ketone or polyether block amide (e.g., PEBAX^{®}). The heat-welding temperature is lower than the glass-transition temperature of the polyether ether ketone or polyether block amide (e.g., PEBAX^{®}) but higher than the glass-transition temperature(s) of the polyurethane(s).

In addition to being coupled to the pump-outlet tube, the inlet guard is fixed over the distal portion of the frame. For example, in some embodiments, the inlet guard is coupled, e.g., bonded, to the distal portion of the frame, e.g., using a combination of heat and pressure as described above, with reference to Fig. 6A, for the coupling of the pump-outlet tube to the frame. Alternatively or additionally, as shown in Fig. 8D, the inlet guard is fixed over distal portion 40 of the frame by fixing proximal flaps 146 at least partly over central portion 38 of the frame. For example, the proximal flaps may be sandwiched between inner lining 39 and pump-outlet tube 24 over the central portion of the frame. Alternatively or additionally, the inlet guard is fixed over the distal portion of the frame by coupling the inlet guard to distal bearing housing 118H and/or to coupling portion 31 of the frame. For example, in some embodiments, multiple distal flaps 148 are formed in sheet 144, and the inlet guard is coupled to the bearing housing and/or to coupling portion 31 by coupling distal flaps 148 to the bearing housing and/or to coupling portion 31.

Reference is now made to Fig. 8C, which is a schematic illustration of sheet 144, in accordance with some embodiments. Reference is also made to Fig. 8D, which is a schematic illustration of frustoconical inlet guard 145, which is formed from sheet 144 as described above with reference to Figs. 8A-B, fixed over distal portion 40 of frame 34, in accordance with some embodiments.

In some embodiments, proximal flaps 146 are shaped such that, when the proximal flaps are fixed at least partly over central portion 38 of the frame, the proximal flaps do not overlap any of struts 37 of the frame. Thus, advantageously, the proximal flaps do not overly interfere with the coupling of the pump-outlet tube to the frame, and do not overly enlarge the diameter of the pump head. For example, the proximal flaps may be shaped such that, when the proximal flaps are fixed at least partly over the central portion of the frame, the proximal flaps fit between struts 37 while abutting the struts. Thus, advantageously, despite not overlapping the struts, the proximal flaps provide a large surface area for the coupling of the inlet guard to the pump-outlet tube.

Alternatively or additionally, proximal flaps 146 are shaped to define multiple flap openings 161, and the pump-outlet tube and the inner lining are heat welded to one another at least partly via flap openings 161. In other words, as the pump-outlet tube is heated during the heat-welding procedure, the pump-outlet tube passes through flap openings 161 and bonds to the inner lining. Typically, the pump-outlet tube also bonds to the inner lining between the proximal flaps.

In some embodiments, at least some flap openings 161, such as those flap openings at a distal portion of the proximal flaps, are sized and shaped similarly to blood-inlet openings 108. One advantage of such embodiments is ease of manufacture. Another advantage is that even if the pump-outlet tube does not completely cover the proximal flaps, the uncovered portion of the flaps does not compromise the functionality of the inlet guard.

In some embodiments, one or more tabs 155 extend from sheet 144, e.g., from a lateral edge 156 of the sheet (as shown in Fig. 8C). Sheet 144 is rolled by pulling tabs 155. Subsequently to rolling the sheet, the tabs are removed (e.g., cut) from the sheet.

It is noted that embodiments described above with reference to Figs. 8A-D are applicable even to cases in which the inlet guard is manufactured from a tube, rather than from a sheet of material. Examples of such embodiments include the sandwiching of the proximal flaps in the heat welding process, the flap openings, the higher glass-transition temperature of the inlet guard, the shaping of the proximal flaps to avoid overlapping the frame struts, and the coupling of the inlet guard (e.g., via the distal flaps) to the distal bearing housing.

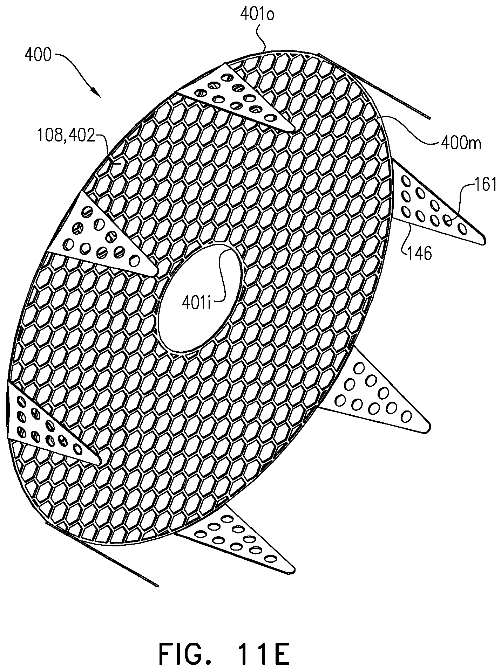

Furthermore, these embodiments are applicable even to cases in which the main body of the inlet guard is flat and is disposed within the frame, as further described below with reference to Figs. 11E-F.

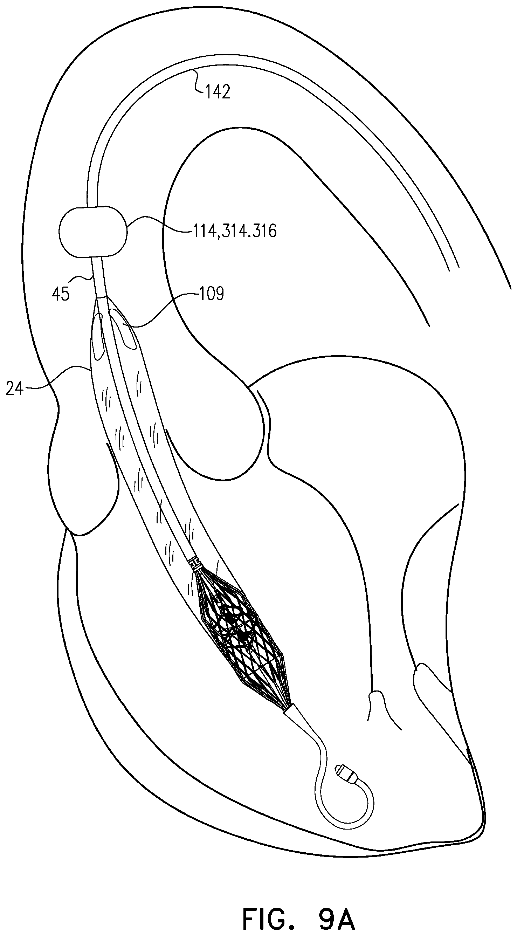

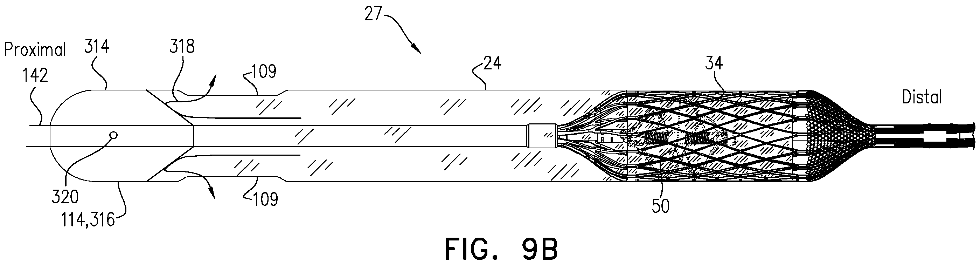

Reference is now made to Fig. 9A, which is a schematic illustration of an expandable element 314 surrounding delivery tube 142, in accordance with some embodiments. Reference is also made to Fig. 9B, which is a schematic illustration of pump-head portion 27, in accordance with some embodiments.

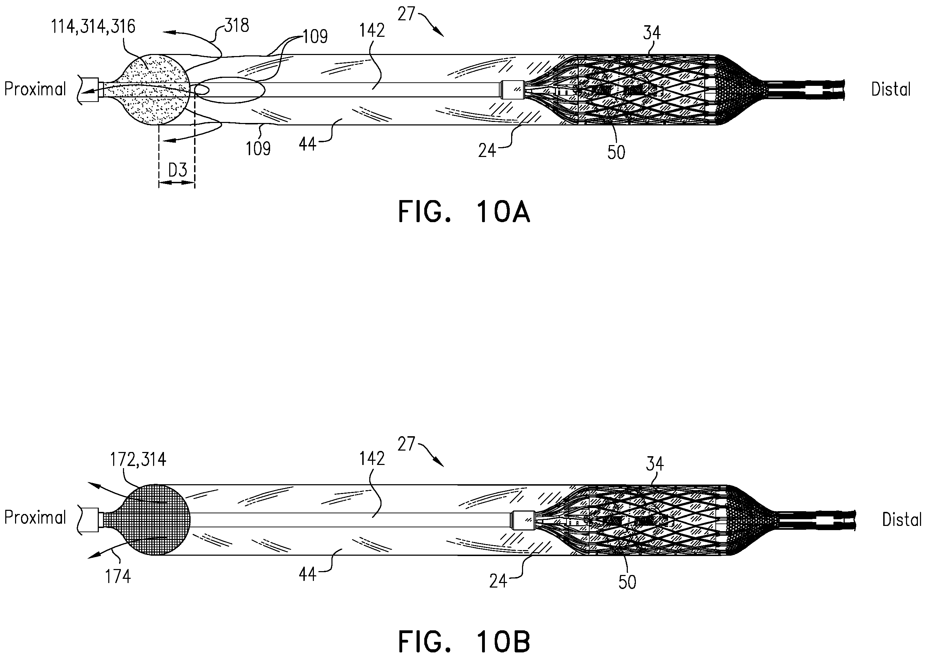

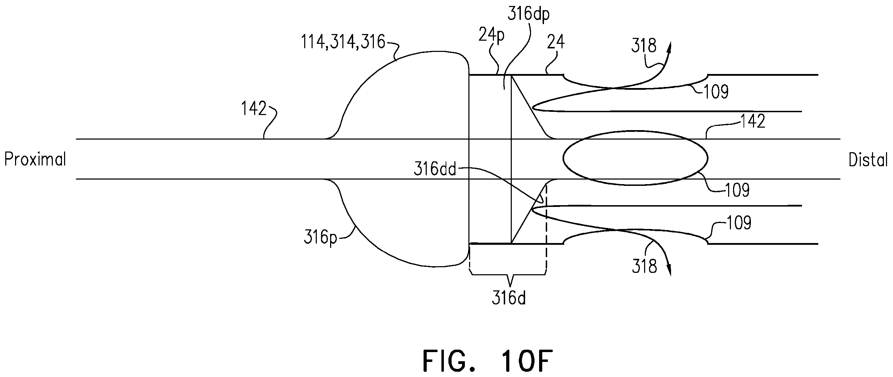

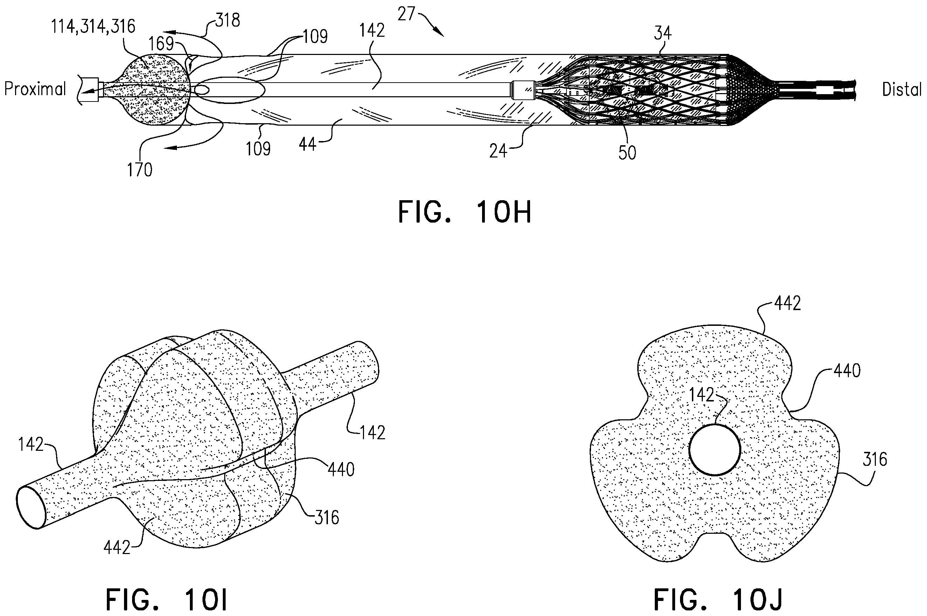

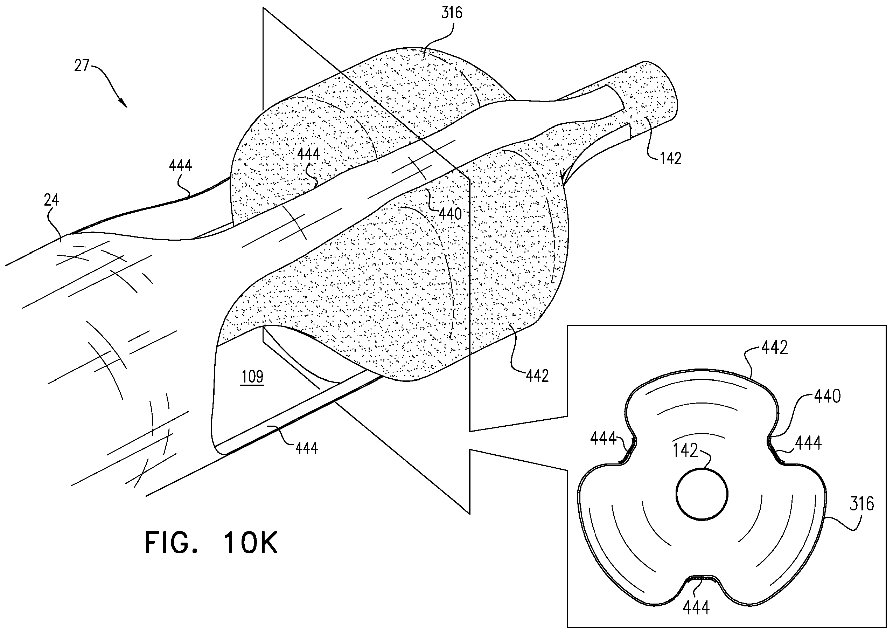

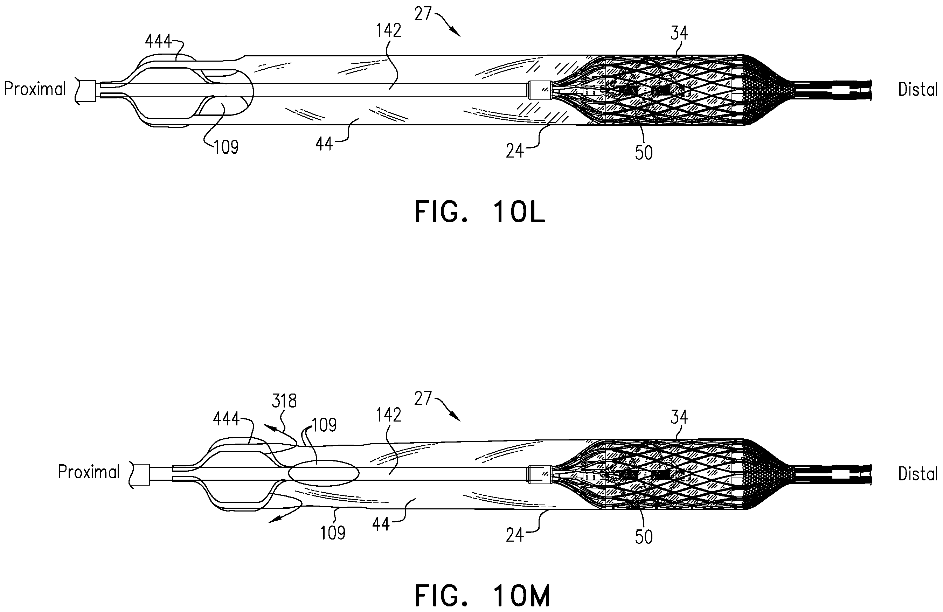

In some embodiments, as shown in Figs. 9A-B (and in Figs. 10A-B, Figs. 10F-H, and Figs. 10K-P, which are described below), expandable element 314 surrounds delivery tube 142. In some such embodiments, expandable element 314 comprises an expandable stent or expandable braided element. Alternatively, expandable element 314 comprises an inflatable element 316 (e.g., a balloon 114). For some applications, inflatable element 316 is inflated using a fluid (e.g., air or saline) that is pumped through the ventricular-assist device. For example, in some embodiments, as shown in Fig. 9B, the wall of the delivery tube is shaped to define one or more openings 320, and inflatable element 316 surrounds openings 320 such that a fluid flowing, via the openings, from the delivery tube into the inflatable element inflates the inflatable element. Typically, the inflating fluid includes purging fluid, which, distally to openings 320, purges the interface between the axial shaft and any stationary bearings (including radial and/or thrust bearings) that don't rotate with the axial shaft. Alternatively or additionally, the inflating fluid comes from a separate, dedicated supply.

Typically, expandable element 314 is proximal to blood-outlet openings 109, with the length of the delivery tube between expandable element 314 and the blood-outlet openings being less than 30 mm.

In some embodiments, as shown in Fig. 9A, expandable element 314 is entirely proximal to the pump-outlet tube. For example, in some embodiments, expandable element 314 is disposed slightly proximally to the interface between delivery tube 142 and pump-outlet tube 24 (as shown in Fig. 9A). For example, in some embodiments, expandable element 314 is slightly proximal to tubular coupling portion 45 or to strips 29 (Fig. 1D).

In other embodiments, as shown in Fig. 9B, expandable element 314, at least when expanded, is disposed at least partly within, e.g., entirely within, pump-outlet tube 24. In some such embodiments, the pump-outlet tube does not comprise a conical proximal portion, and is not coupled directly to delivery tube 142.

Expandable element 314 is configured to protect the aortic wall from injury, e.g., by inhibiting the edges of blood-outlet openings 109, which are sometimes sharp, from contacting the wall of the aorta. Alternatively or additionally, expandable element 314 is configured to center a portion of the ventricular assist device (e.g., the portion of delivery tube 142 near the pump-outlet tube) within the aorta. Expandable element 314 is configured to perform these functions by abutting the aortic wall.

Alternatively or additionally, expandable element 314 is shaped to direct the blood through blood-outlet openings 109, as indicated in Fig. 9B by blood-flow arrows 318. For example, in some embodiments, the distal end of the expandable element has a width that decreases moving distally, e.g., the distal end is frustoconical, such that the blood is directed by the distal end of the expandable element, at an angle, through the blood-outlet openings. Alternatively or additionally, the expandable element has an angled and/or a curved surface that is configured to direct the blood flow in this manner. For some applications, by directing blood flow in this manner, the overall pumping efficiency of the device is increased, relative to if the device would not include an expandable element.

It is noted that expandable element 314 may be combined with any of the embodiments of pump-outlet tube 24 described with reference to Figs. 33A-C of WO 24/057252 to Tuval, which is incorporated herein by reference.

In some applications, a ventricular assist device that comprises expandable element 314 is selected for longer-term treatments, such as treatment for cardiogenic shock, due to the protection provided by the expandable element. On the other hand, for shorter-term treatments, such as a percutaneous coronary intervention, a device without expandable element 314, e.g., as shown in Fig. 1D, is selected, given that over shorter periods, there is less chance of injury to the aortic wall. Alternatively or additionally, other factors are considered when deciding whether to select a device that comprises expandable element 314.

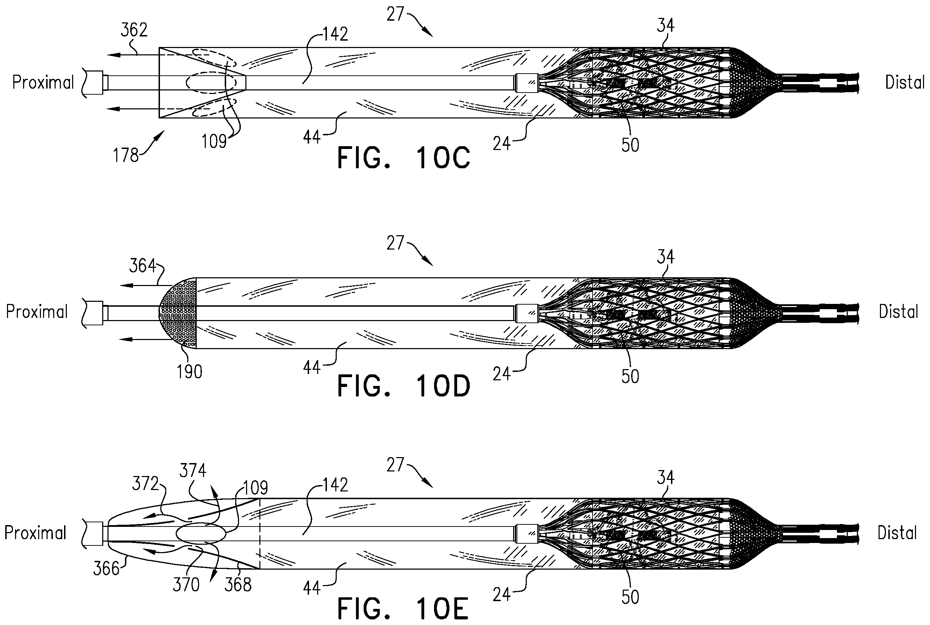

Reference is now made to Figs. 10A, 10B, 10C, and 10D, which are schematic illustrations of pump-head portion 27, in accordance with some embodiments.

Fig. 10A is similar to Fig. 9B, in that Fig. 10A shows expandable element 314, which comprises an inflatable element 316 (e.g., a balloon 114), surrounding delivery tube 142 and disposed at least partly (e.g., entirely) within pump-outlet tube 24, proximally to the blood-outlet openings. Furthermore, in Fig. 10A, as in Fig. 9B, the expandable element acts as a blood flow director, by directing blood from the proximal end of the pump-outlet tube through the blood outlet openings, as indicated by blood-flow arrows 318. Fig. 10A differs from Fig. 9B, however, in the shape of the expandable element; in particular, in Fig. 10A, the expandable element is more spherically-shaped.

In some embodiments, regardless of the shape of the expandable element, the expandable element is coupled to the lateral wall of the pump-outlet tube, which is shaped to define blood-outlet openings 109, within 0.5-5 mm (e.g., within 1-3 mm) of the blood-outlet openings. In other words, in some embodiments, the axial distance D3 between the distal-most portion of the lateral wall that is coupled to the expandable element and the proximal-most portion of the edge of each of the blood-outlet openings is between 0.5 and 5 mm (e.g., within 1 and 3 mm). Advantageously, this range is small enough such that any blood directed away from the expandable element can quickly exit the blood-outlet openings, yet is large enough such that the expandable element does not push the edges of the blood-outlet openings, which are sometimes sharp, into the wall of the aorta.

Referring to Fig. 10B, for some applications, expandable element 314 is a porous expandable element, such as an expandable cage or stent 172, that surrounds delivery tube 142 within, and/or immediately proximally to, the pump-outlet tube, such that the blood is pumped through the porous expandable element. For example, in some embodiments, the porous expandable element is disposed at the proximal end of the pump-outlet tube, and the proximal end of the pump outlet tube is coupled to delivery tube 142 via the porous expandable element. For some such applications, the pump-outlet tube does not define blood-outlet openings 109 (Fig. 10A). Rather, blood flows out of the pump-outlet tube exclusively via the porous expandable element, as indicated by blood-flow arrows 174. For some applications, the porous expandable element comprises a structure made of a shape-memory alloy, such as a laser-cut shape-memory alloy and/or a braided shape-memory alloy.

Referring to Fig. 10C, for some applications, a proximal portion 178 of pump-outlet tube 24, which defines blood-outlet openings 109, is folded inwardly toward the distal end of the pump-outlet tube. Typically, as shown, proximal portion 178 is folded inwardly such that the blood-outlet openings direct blood proximally (substantially parallel to the axis of outer tube 142) rather than radially outwardly (away from the axis of outer tube 142), as indicated by blood-flow arrows 362. For some applications, proximal portion 178 is folded inwardly such that the blood-outlet tube forms a protective layer between blood flowing out of the blood-outlet openings and the walls of the subject's aorta.

Referring to Fig. 10D, for some applications, the blood-outlet openings 109 are defined by a substantially proximally-facing surface 190 of the pump-outlet tube, rather than being defined by a lateral surface of the pump-outlet tube. Typically, for such applications, blood flow from the pump-outlet tube is axially directed, as indicated by blood-flow arrows 364.

Reference is now made to Fig. 10E, which is a schematic illustration of pump-outlet tube 24 that defines a blood-flow chamber 366 at its proximal end, in accordance with some embodiments. For some applications, the blood-flow chamber is defined by an internal membrane 368 that is disposed within the proximal end of the pump-outlet tube and that defines holes 370 therethrough. Blood flows into the blood-flow chamber via holes 370, as indicated by blood-flow arrows 372. Subsequently, the blood flows out of the blood-flow chamber and into the subject's aorta via blood-outlet openings 109 (which are generally as described hereinabove), as indicated by blood-flow arrows 374. Typically, by virtue of the blood flowing through the blood-flow chamber, the blood-flow chamber inflates such as to center a portion of the left-ventricular assist device (e.g., the delivery tube, and in particular, the portion of the delivery tube near pump-outlet tube 24) within the aorta, by contacting the aorta wall. Typically, the internal membrane is shaped so as to direct the blood flow out of the blood-outlet openings.

For some applications, internal membrane 368 is a continuation of pump-outlet tube 24, and the internal membrane is covered with an external membrane, which defines the blood-outlet openings, and which forms the external surface of blood-flow chamber 366. For such applications, the proximal end of the blood-outlet tube is shaped so as to direct the blood flow out of the blood-outlet openings.

Typically, the combination of the proximal end of the blood-outlet tube and an additional membrane (whether an internal membrane or an external membrane) is configured to define blood-flow chamber 366, which typically functions as described above. In general, the scope of the present disclosure includes any structure that provides a blood-flow chamber disposed at a proximal end of the pump-outlet tube, the blood-flow chamber defining (a) holes 370 via which blood is pumped into the blood-flow chamber and (b) blood-outlet openings 109 configured to be disposed within the aorta, via which the blood flows out of the blood-flow chamber and into the aorta.

Reference is now made to Fig. 10F, which is a schematic illustration of inflatable element 316, such as balloon 114, disposed at the proximal end of pump-outlet tube 24, in accordance with some embodiments.

Fig. 10F is similar to Fig. 9B with respect to the features noted above with reference to Fig. 10A, but differs with respect to the shape of inflatable element 316. In particular, in Fig. 10F, inflatable element 316 comprises two portions: a distal inflatable-element portion 316d, which is coupled to the inside of the lateral wall of pump-outlet tube 24 (thus, typically, sealing the proximal end of the pump-outlet tube), and a proximal inflatable-element portion 316p, which is wider than the pump-outlet tube and is disposed proximally to the pump-outlet tube.

In general, it is desired to avoid contact between the wall of the aorta and the portion 24p of the lateral wall of the pump-outlet tube at which the pump-outlet tube is coupled to the inflatable element. Proximal inflatable-element portion 316p is configured to protect the aortic wall from such contact. Furthermore, typically, proximal inflatable-element portion 316p is configured to center delivery tube 142 within the aorta.

Typically, distal inflatable-element portion 316d is at least partly cylindrical, to facilitate coupling the distal inflatable-element portion to the pump-outlet tube. For example, in some embodiments, a proximal portion 316dp of distal inflatable-element portion 316d is cylindrical. Alternatively or additionally, the distal inflatable-element portion is shaped to direct the blood through blood-outlet openings 109, as indicated by blood-flow arrows 318. For example, in some embodiments, a distal portion 316dd of distal inflatable-element portion 316d has a width that decreases moving distally, e.g., distal portion 316dd is frustoconical, such that distal portion 316dd directs the blood.

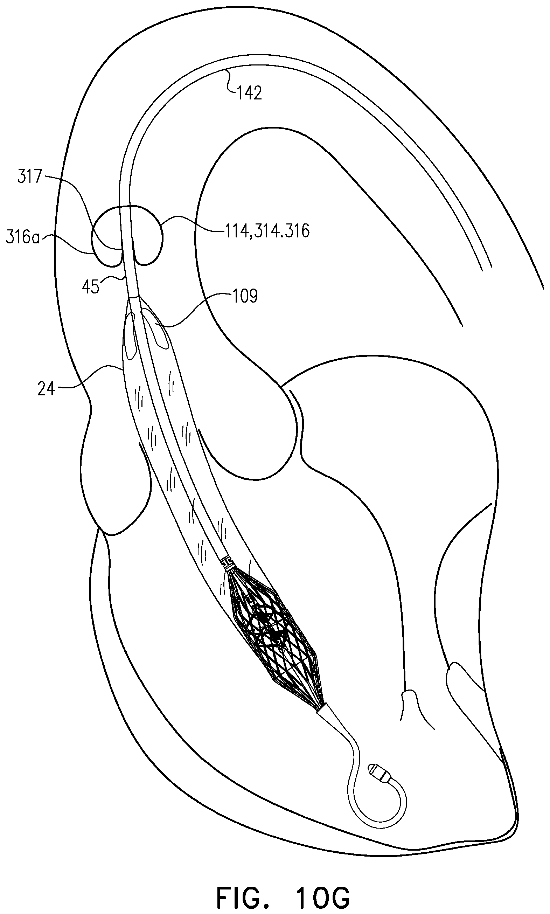

Reference is now made to Fig. 10G, which is a schematic illustration of inflatable element 316, such as balloon 114, disposed proximally to pump-outlet tube 24, in accordance with some embodiments.

Fig. 10G is similar to Fig. 9A. A difference, however, is that in Fig. 10G, a distal portion 316a of the inflatable element is everted inwardly and is coupled to delivery tube 142 at an interface 317. Thus, advantageously, the coupling of the pump-outlet tube to the delivery tube does not interfere with the coupling of the inflatable element to the delivery tube.

In some embodiments, as shown in Fig. 10G, the pump-outlet tube comprises tubular coupling portion 45, via which the pump-outlet tube is coupled to delivery tube 142. Distal portion 316a of the inflatable element is coupled to the delivery tube proximally to the tubular coupling portion, e.g., at a distance of less than 20 mm, such as less than 10 mm, from the tubular coupling portion.

Reference is now made to Fig. 10H, which is a schematic illustration of pump-head portion 27, in accordance with some embodiments. Fig. 10H is similar to Fig. 9B with respect to the features noted above with reference to Fig. 10A.

In some embodiments, to form blood-outlet openings 109, portions of the lateral wall of pump-outlet tube 24 are cut. In some such embodiments, rather than removing these portions (i.e., rather than cutting closed curves in the lateral wall such that the portions are completely detached from the rest of the wall), flaps 170 are cut in the lateral wall (i.e., open curves are cut in the lateral wall so as to define flaps 170). Flaps 170 are then folded inwardly, as indicated by folding indicators 169, and coupled to the inflatable element.

As noted above with reference to Fig. 9A, the edges of the blood-outlet openings are sometimes sharp, and there is a risk of these edges contacting the wall of the aorta. Inflatable element 316 can mitigate this risk by abutting the aortic wall, thereby keeping the edges at a distance from the wall. However, there is still some risk that, in certain situations, the inflatable element will push the edges into the wall. One way to mitigate this risk is to distance the inflatable element from edges, as described above with reference to Fig. 10A. Alternatively or additionally, flaps 170 mitigate this risk by eliminating the portions of the edges adjacent to the inflatable element, e.g., using the technique described with reference to Fig. 10H.

Reference is now made to Figs. 10I-J, which are schematic illustrations of inflatable element 316 from oblique and frontal perspectives, respectively, in accordance with some embodiments.

In some embodiments, inflatable element 316 is shaped to define one or more (e.g., three, four, or 6-10) grooves 440, the function of which is described with reference to subsequent figures. Typically, by virtue of being shaped to define grooves 440, inflatable element 316 comprises lobes 442, the number of lobes 442 being the same as the number of grooves. Grooves 440 run between lobes 442.

Reference is now made to Figs. 10K-M, which are schematic illustrations of pump-head portion 27, in accordance with some embodiments.

By way of introduction, it is noted that to couple pump-outlet tube 24 to inflatable element 316, an adhesive is typically required. However, the adhesive may cause the inflatable element to become less flexible and/or injure the aortic wall in some cases. Hence, in some embodiments, the pump-outlet tube is coupled (e.g., heat welded) to delivery tube 142, rather than to the inflatable element. In such embodiments, pump-outlet tube 24 comprises multiple tabs 444, which extend proximally from the proximal portion of the pump-outlet tube, and which are coupled to the delivery tube proximally to the inflatable element.

In some embodiments, as shown in Figs. 10K-M, tabs 444 pass through grooves 440. Advantageously, the grooves reduce the profile of the device, such that the tabs do not contact the aortic wall. Furthermore, the grooves hold the tabs in place, thereby reducing the risk of blood stagnating between the tabs and the inflatable element. Typically, the tabs are coupled to the delivery tube immediately proximally to the inflatable element, thereby further reducing the risk of stagnant blood.

In some embodiments, as shown in Figs. 10K-L, inflatable element 316 is offset proximally from the proximal portion of the pump-outlet tube so as to define multiple blood-outlet openings 109 between tabs 444 and between the proximal portion of the pump-outlet tube and the inflatable element. The blood exits the proximal portion of the pump-outlet tube via these blood-outlet openings. Advantageously, it is typically not necessary to cut separate blood-outlet openings in the lateral wall of the pump-outlet tube.

In other embodiments, as shown in Fig. 10M, the inflatable element contacts (e.g., is coupled to) the proximal portion of the blood-outlet tube, and the blood exits via blood-outlet openings 109 in the lateral wall of the pump-outlet tube.

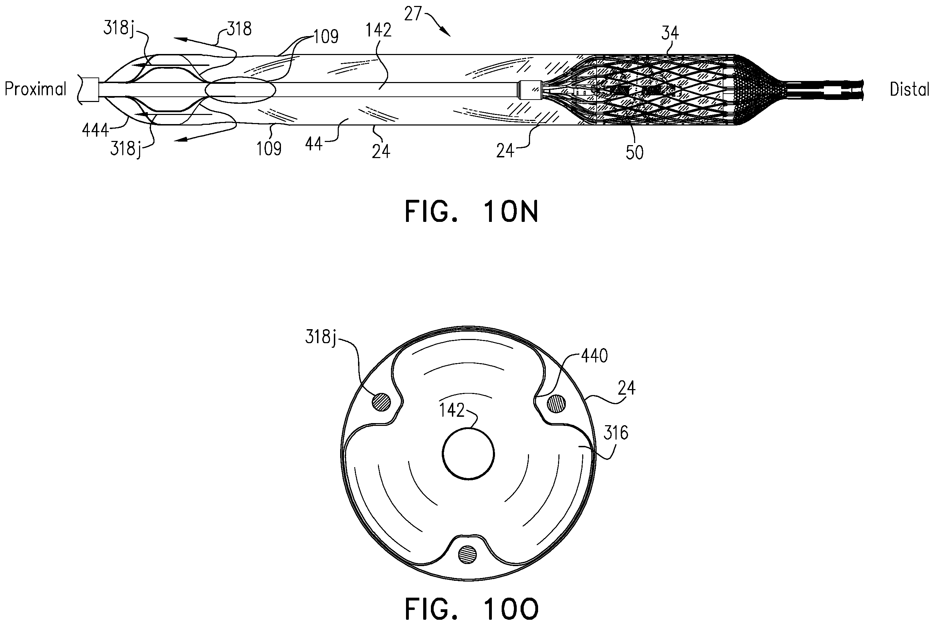

Reference is now made to Fig. 10N, which is a schematic illustration of pump-head portion 27, in accordance with some embodiments. Reference is also made to Fig. 10O, which shows a schematic frontal view of the proximal end of pump-head portion 27, in accordance with some embodiments.

Figs. 10N-O show an alternate solution for reducing the risk of stagnant blood. In particular, inflatable element 316 is disposed within the proximal portion of the pump-outlet tube, such that tabs 444 are mostly or entirely proximal to the inflatable element. Typically, the tabs are coupled to delivery tube 142 between 0.5 and 6 mm, e.g., 1-3 mm, from the inflatable element.

As indicated by blood-flow arrows 318j, at least some of the blood exits the proximal portion of the pump-outlet tube via grooves 440. These jets of blood help reduce the risk of blood stagnating between the pump-outlet tube and the inflatable element or between the tabs and the delivery tube.

In some embodiments, as shown in Fig. 10N, the lateral wall of the pump-outlet tube is shaped to define blood-outlet openings 309, and some of the blood exits the proximal portion of the pump-outlet tube via the blood-outlet openings. In other embodiments, all the blood exits via grooves 440.

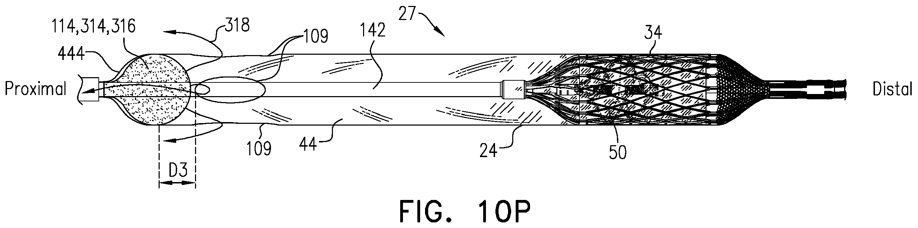

Reference is now made to Fig. 10P, which is a schematic illustration of pump-head portion 27, in accordance with some embodiments.

The embodiment shown in Fig. 10P is similar to that shown in Fig. 10A. For example, in Fig. 10P, the lateral wall of pump-outlet tube is shaped to define blood-outlet openings 109, and inflatable element 316 surrounds delivery tube 142 and is disposed at least partly (e.g., entirely) within the pump-outlet tube proximally to the blood-outlet openings. However, in Fig. 10P, multiple tabs 444 extend proximally from the proximal portion of the pump-outlet tube and are coupled to the delivery tube proximally (e.g., immediately proximally) to the inflatable element. Thus, advantageously, even if some blood flows to the proximal side of inflatable element 316 (instead of exiting the pump-outlet tube via blood-outlet openings 109), this blood can exit the pump-outlet tube via the spaces between tabs 444.

In some embodiments, as shown in Fig. 10P, the inflatable element is not shaped to define any grooves. In other embodiments, the inflatable element is shaped to define one or more grooves and the tabs pass through the grooves, e.g., as shown in Fig. 10M.

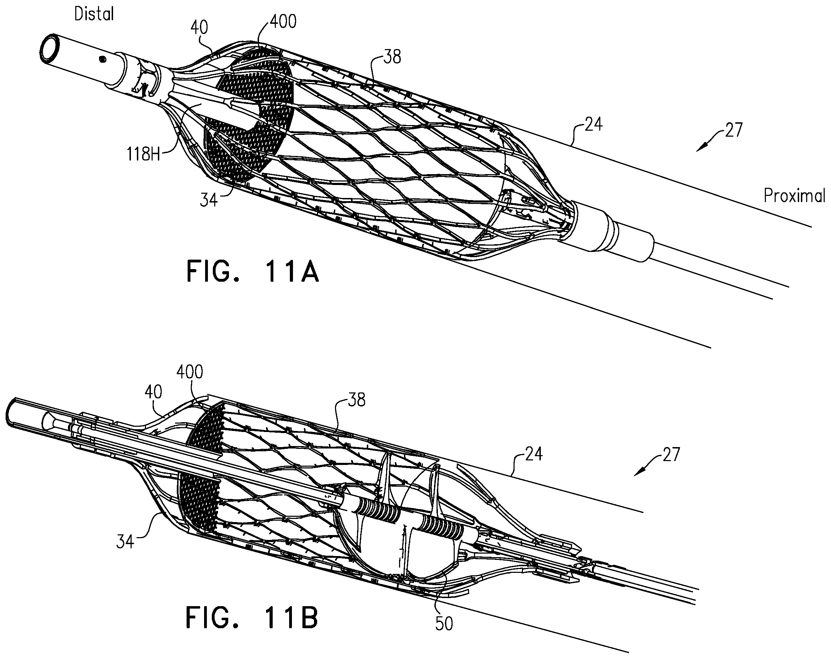

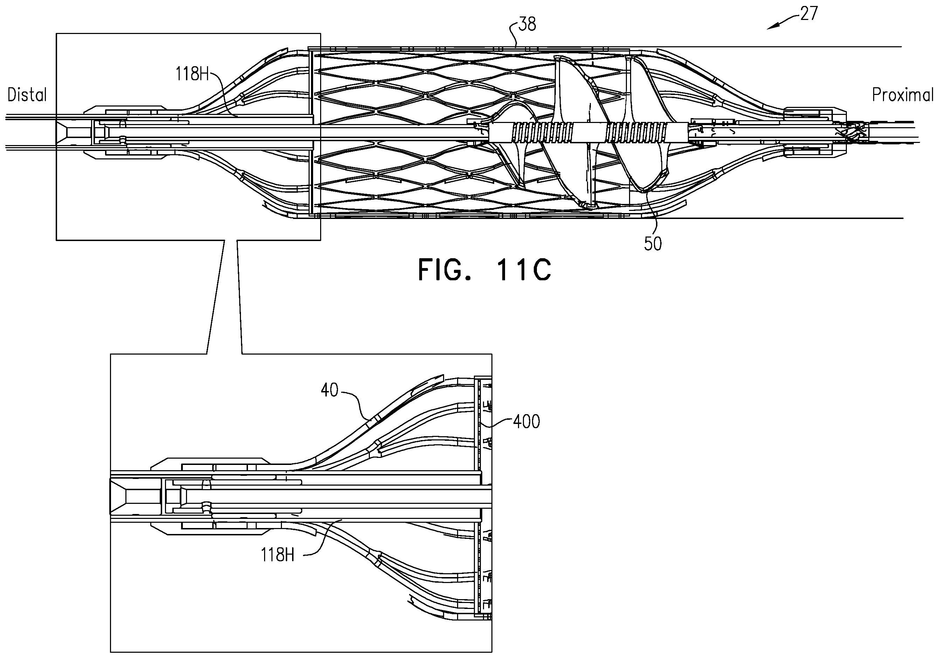

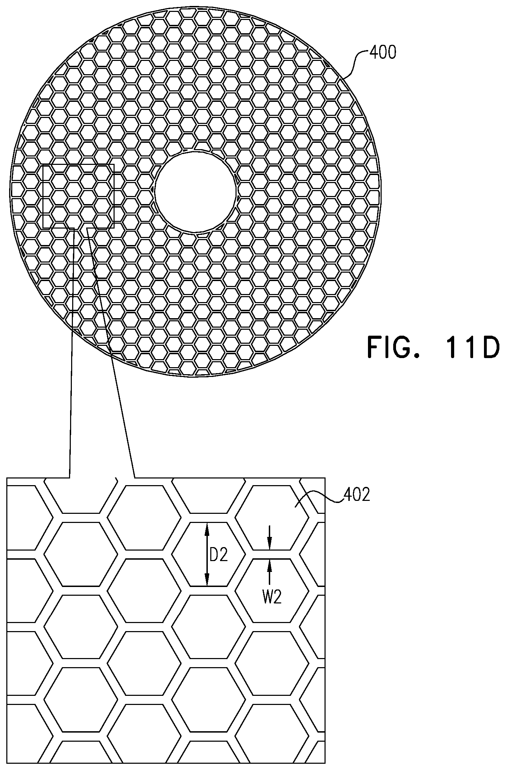

Reference is now made to Figs. 11A, 11B, 11C, and 11D, which are schematic illustrations of portions of ventricular assist device 20, the device including an inlet guard 400 disposed inside frame 34, in accordance with some embodiments. Inlet guard 400 is shaped to define one or more holes 402, shown enlarged in Fig. 11D, which are disposed around the axial shaft and within frame 34 distally to the impeller, such that the blood flows to the impeller via holes 402. The inlet guard may be coupled to the struts of frame 34, to inner lining 39 (Fig. 4) of the frame, to the inner wall of pump-outlet tube 24, and/or to distal bearing housing 118H.

For some applications, inlet guard 400 is flat and/or is disposed such that it is perpendicular to the axial shaft (i.e., to the longitudinal axis of the frame). Thus, advantageously, the inlet guard may occupy relatively little space, and/or may provide an advantageous flow direction for the blood. Typically, the inlet guard is toric.

For some applications, the ventricular assist device includes a thrust bearing in pump-head portion 27. Typically, for such applications, the impeller does not move distally of cylindrical portion 38 of frame 34 (either during delivery of the device to the left ventricle or during operation of the device).

For some applications, the inlet guard is placed within the frame at the distal end of the central cylindrical portion of the frame or in the vicinity thereof, e.g., within 1 mm of the distal end of the cylindrical portion. This placement may simplify the assembly of the blood pump. For some applications, distal bearing housing 118H extends into the distal conical portion of the frame (e.g., until at least the end of the central cylindrical portion of the frame) and the inner edge of inlet guard 400 is couple to the distal bearing housing, as shown in Figs. 11A-B.

Typically, the inlet guard is polymeric, i.e., is made of a polymeric material (such as a polyurethane (e.g., Pellethane^{®}), polyethylene terephthalate ("PET"), ultra-high-molecular-weight polyethylene ("UHMWPE"), and/or polyether block amide (e.g., Pebax^{®})) that is shaped to define holes 402. For some applications, the thickness of the inlet guard is more than 40 microns (e.g., more than 50 microns), and/or less than 100 microns (e.g., less than 80 microns), for example, 40-100 microns or 50-80 microns. Thus, the inlet guard may be configured to withstand pressure yet be crimpable.

Typically, for applications in which ventricular assist device 20 includes inlet guard 400 disposed inside frame 34, pump-outlet tube 24 does not extend until the distal end of distal conical portion 40 of frame 34. Moreover, pump-outlet tube 24 may have an open distal end, rather than terminating in a distal conical portion. (Thus, the inlet guard may simplify the manufacture of the blood pump.) The distal end of the pump-outlet tube may be proximal to the distal end of the distal conical portion of the frame. For example, the distal end of the pump-outlet tube may be within 1 mm of the distal end of the central cylindrical portion of frame 34, i.e., the pump-outlet tube may extend only until the end of the cylindrical portion of frame 34, or the vicinity thereof. Blood may thus flow into frame 34 via openings defined by the distal conical portion of the frame.

For some applications, holes 402 of inlet guard 400 are sized such as (a) to allow blood to flow from the subject's left ventricle into pump-outlet tube 24 and (b) to block structures from the subject's left ventricle from entering into the pump-outlet tube. Typically, for such applications, the inlet guard is configured to reduce a risk of structures from the left ventricle (such as chordae tendineae, trabeculae carneae, and/or papillary muscles) entering into pump-outlet tube 24 and potentially being damaged by the impeller and/or the axial shaft, and/or causing damage to the ventricular assist device.

For some applications, inlet guard 400 defines more than 10 holes, more than 50 holes, more than 100 holes, more than 150 holes, or more than 200 holes e.g., 50-100 holes, 100-150 holes, 150-200, or 200-300 holes. For some applications, the holes are sized such as (a) to allow blood to flow from the subject's left ventricle into the tube and (b) to block structures from the subject's left ventricle from entering into the frame. Typically, for such applications, the inlet guard is configured to reduce a risk of structures from the left ventricle (such as chordae tendineae, trabeculae carneae, and/or papillary muscles) entering into the cylindrical portion of frame 34 and potentially being damaged by the impeller and/or the axial shaft, and/or causing damage to the left ventricular assist device. Therefore, for some applications, the holes are shaped such that, for each of the holes, the span of the hole in at least one direction is less than 1 mm, e.g., 0.1-1 mm, or 0.2-0.6 mm. By defining such a small width (or span), it is typically the case that structures from the left ventricle (such as chordae tendineae, trabeculae carneae, and/or papillary muscles) are blocked from entering the cylindrical portion of frame 34.

For some applications, each of the holes defines an area of more than 0.05 square mm (e.g., more than 0.1 or 0.3 square mm), and/or less than 5 square mm (e.g., less than 3 or 1 square mm), e.g., 0.05-5, 0.05-3, 0.1-1, 0.1-5, or 0.3-1 square mm.

Typically, the inlet guard has a porosity of at least 40 percent, e.g., more than 50 percent, more than 60 percent, or more than 70 percent (where porosity is defined as the percentage of the area of this portion that is porous to blood flow). Thus, on the one hand, the holes are relatively small (in order to prevent structures of the left ventricular from entering the frame), but on the other hand, the porosity of the portion of the pump-outlet tube that defines the holes is relatively high, such as to allow sufficient blood flow into the pump-outlet tube.

For some applications, each of the holes has a circular or a polygonal shape. For some applications, each of the holes has a hexagonal shape, as shown most clearly in Fig. 11D. Typically, using openings having a hexagonal shape allows the inlet guard to have a relatively high porosity (e.g., as described hereinabove), while providing the inlet guard with sufficient material between the holes to prevent tearing and/or stretching of the material.

As shown in Fig. 11D, for some applications, a width W2 of gaps between adjacent holes 402 (i.e., the distance between each pair of adjacent holes) is more than 0.01 mm (e.g., more than 0.02 mm), and/or less than 0.2 mm (e.g., less than 0.15 mm), for example, 0.01-0.2 mm, or 0.02-0.15 mm.

As further shown in Fig. 11D, for some applications, the distance D2 between opposing sides of each of the hexagons (or other types of polygons) is more than 0.1 mm (e.g., more than 0.2 mm) and/or less than 0.8 mm (e.g., less than 0.6 mm), e.g., 0.1-0.8 mm, or 0.2-0.6 mm. Typically each of the polygons encloses a circle (such that any structure that cannot pass through such a circle would be unable to pass through the polygon). Typically, the diameter of the circle enclosed by the polygon is the equivalent of distance D2, e.g., more than 0.1 mm (e.g., more than 0.2 mm) and/or less than 0.8 mm (e.g., less than 0.6 mm), e.g., 0.1-0.8 mm, or 0.2-0.6 mm.

For some applications, the frame is assembled with the inlet guard inside in the following manner. As described hereinabove, during assembly of the pump-head portion, the proximal end of frame 34 is typically open. For some applications, the inlet guard is placed through the open proximal end of the frame while being supported upon a rod (e.g., a mandrel). The inlet guard typically has an overall torus shape, with the edges of the shape defining inner and outer circles, as shown in Fig. 11D. The inner circle defined by the inlet guard is typically coupled to distal bearing housing 118H, as shown in Figs. 11A-B and the outer circle is coupled to struts of frame 34, to pump-outlet tube 24, and/or to inner lining 39. For some applications, the aforementioned coupling of the inlet guard to other portions of the device is performed via suturing, via hooks, via adhesive, and/or via heat fusion.

As noted above, in some embodiments, the inlet guard is coupled to the distal bearing housing, which may house a radial and/or thrust bearing. In such embodiments, typically, the distal bearing housing is partly or entirely disposed within frame 34. For example, at least 10 percent, 50 percent, or 80 percent of the length of the bearing housing may be disposed within the frame. Moreover, the distal bearing housing may extend into the frame even for applications in which the blood pump does not comprise inlet guard 400.

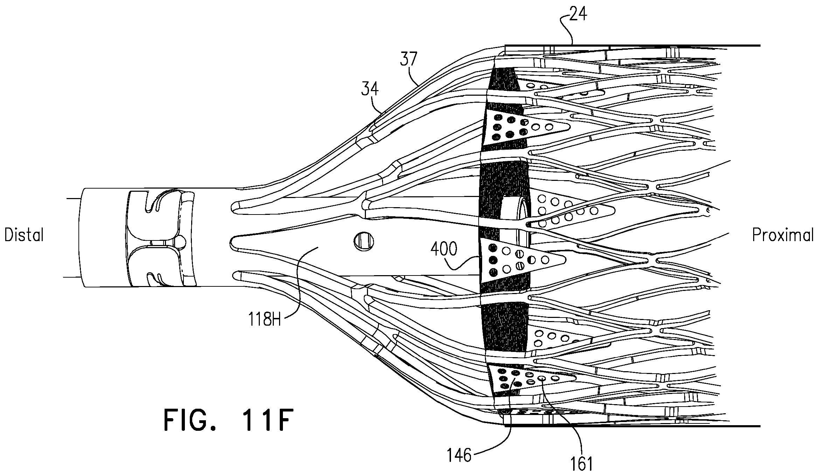

Reference is now made to Fig. 11E, which is a schematic illustration of inlet guard 400, in accordance with some embodiments. Reference is also made to Fig. 11F, which is a schematic illustration of inlet guard 400 disposed within frame 34, in accordance with some embodiments. (It is noted that some elements of the pump-head portion, such as axial shaft 92 and inner lining 39, are not shown in Fig. 11F.)

The embodiment of inlet guard 400 shown in Figs. 11E-F combines aspects of the embodiments of Figs. 11A-D with those of Figs. 8A-D. In particular, the main body 400m of the inlet guard, which is shaped to define holes 402 (which can alternatively be referred to as blood-inlet openings 108) is flat and is disposed within frame 34, e.g., such that main body 400m is perpendicular to axial shaft 92, as in Figs. 11A-D. For example, main body 400m may be toric, comprising an inner circular edge 401i, which is optionally coupled to the distal bearing housing, and an outer circular edge 401o, which is optionally coupled to struts 37 of frame 34, to pump-outlet tube 24, and/or to inner lining 39. In addition, as in Figs. 8A-D, the inlet guard comprises proximal flaps 146, via which the inlet guard is coupled to pump-outlet tube 24 and/or to the inner lining of the frame. For example, the proximal flaps may be sandwiched between the pump-outlet tube and the inner lining, e.g., via the heat welding process described above. In some embodiments (not shown), inlet guard 400 includes distal flaps 148 (e.g., as shown in Figs. 8A-D), which extend distally from inner edge 401i and are coupled to the distal bearing housing.

As a specific example, for some applications, the inner lining and the pump-outlet tube are heat welded to one another, e.g., as described above with reference to Figs. 6A-B, while the proximal flaps are between the inner lining and the pump-outlet tube. In some such embodiments, to protect inlet guard 400 from degradation during the heat-welding process, the heat-welding temperature (to which the inner lining and the pump-outlet tube are heated) is lower than the glass-transition temperature of the inlet guard but higher than the glass-transition temperature of at least one of the inner lining and the pump-outlet tube (and in some embodiments, higher than the glass-transition temperature of both of the inner lining and the pump-outlet tube). Furthermore, as described above, the heat-welding temperature is typically lower than the respective melting points of the inner lining and the pump-outlet tube, such that the pump-outlet tube is bonded to the inner lining without deformation of the inner lining or pump-outlet tube.

In some embodiments, inlet guard 400 is made of the same material as the inner lining and/or the pump-outlet tube, such as a polyurethane (e.g., Pellethane^{®}) or a polyether ether ketone. In other embodiments, inlet guard 400 is made of a different material. In some such embodiments, the glass-transition temperature of the material of which the inlet guard is made is higher than the respective glass-transition temperatures of each of the inner lining and the pump-outlet tube. The heat-welding temperature is lower than the glass-transition temperature of the material but higher than the respective glass-transition temperatures of each of the inner lining and the pump-outlet tube.

For example, in some applications, the inner lining is made of a polyurethane (e.g., Pellethane^{®}), the pump-outlet tube is made of polyether block amide (e.g., PEBAX^{®}), and inlet guard 400 is made of a polyether ether ketone. The heat-welding temperature is lower than the glass-transition temperature of the polyether ether ketone but higher than the respective glass-transition temperatures of each of the polyurethane and polyether block amide. Alternatively, the inner lining and pump-outlet tube are made of the same type or different types of polyurethane, and inlet guard 400 is made of a polyether ether ketone or polyether block amide (e.g., PEBAX^{®}). The heat-welding temperature is lower than the glass-transition temperature of the polyether ether ketone or polyether block amide (e.g., PEBAX^{®}) but higher than the glass-transition temperature(s) of the polyurethane(s).

For some applications, inlet guard 400 is coupled to pump-outlet tube 24 and/or to the inner lining of the frame by fixing proximal flaps 146 at least partly over central portion 38 of the frame. For example, the proximal flaps may be sandwiched between inner lining 39 and pump-outlet tube 24 over the central portion of the frame.

In some embodiments, proximal flaps 146 are shaped such that, when the proximal flaps are fixed at least partly over central portion 38 of the frame, the proximal flaps do not overlap any of struts 37 of the frame. Thus, advantageously, the proximal flaps do not overly interfere with the coupling of the pump-outlet tube to the frame, and do not overly enlarge the diameter of the pump head. For example, the proximal flaps may be shaped such that, when the proximal flaps are fixed at least partly over the central portion of the frame, the proximal flaps fit between struts 37 while abutting the struts. Thus, advantageously, despite not overlapping the struts, the proximal flaps provide a large surface area for the coupling of the inlet guard to the pump-outlet tube.

Alternatively or additionally, proximal flaps 146 are shaped to define multiple flap openings 161, and the pump-outlet tube and the inner lining are heat welded to one another at least partly via flap openings 161. In other words, as the pump-outlet tube is heated during the heat-welding procedure, the pump-outlet tube passes through flap openings 161 and bonds to the inner lining. Typically, the pump-outlet tube also bonds to the inner lining between the proximal flaps.

It will be appreciated by persons skilled in the art that the present disclosure is not limited to what has been particularly shown and described hereinabove. Rather, the scope of the present disclosure includes both combinations and subcombinations of the various features described hereinabove, as well as variations and modifications thereof that are not in the prior art, which would occur to persons skilled in the art upon reading the foregoing description.

## Claims

1. An apparatus, comprising:
a blood pump (20), comprising:
a frame (34) comprising a proximal portion (36), a central portion (38) comprising multiple struts (37), and a distal portion (40);
an impeller (50), which is configured to pump blood of a subject proximally, disposed within the frame (34);
a pump-outlet tube (24), which is fixed over the proximal portion (36) of the frame (34) and at least part of the central portion (38) of the frame (34); and
an inlet guard (145), which:
is distal to the impeller (50),
comprises a main body shaped to define one or more blood-inlet openings (108) configured to allow passage of the blood therethrough, and
comprises multiple proximal flaps (146), which are coupled to the pump-outlet tube (24) and are fixed at least partly over the central portion (38) of the frame (34) without overlapping any of the struts (37).

2. The apparatus according to claim 1, wherein the proximal flaps (145) fit between the struts (37) while abutting the struts (37).

3. The apparatus according to claim 1, wherein;
the pump-outlet tube (24) is configured to traverse an aortic valve (26) of the subject, and wherein the impeller (50) is configured to pump the blood from a left ventricle (22) of a heart of the subject, through the pump-outlet tube (24), into an aorta (30) of the subject; or
the blood-inlet openings (108) are sized such that the inlet guard (145) is configured to block chordae tendineae, trabeculae carneae, and papillary muscles of a left ventricle (22) of a heart of the subject from entering the frame (34).

4. The apparatus according to claim 1, wherein:
for each of the blood-inlet openings (108), a span of the blood-inlet opening (108) in at least one direction is less than 1 mm;
an area of each of the blood-inlet openings (108) is 0.05-5 mm²;
a porosity of the inlet guard (145) is at least 40 percent;
each of the blood-inlet openings (108) is hexagonal; or
a distance between each pair of adjacent ones of the blood-inlet openings (108) is 0.01-0.1 mm.

5. The apparatus according to claim 1, wherein the inlet guard (145) is coupled to the distal portion (40) of the frame (34).

6. The apparatus according to any one of claims 1-5, further comprising an inner lining (39) that lines at least part of the central portion (38) of the frame (34) and is coupled to the inlet guard (145).

7. The apparatus according to claim 6, wherein the proximal flaps (146) are coupled to the inner lining (39).

8. The apparatus according to claim 6, wherein the pump-outlet tube (24) and the inner lining (39) are heat welded to one another with the proximal flaps (146) being disposed between the pump-outlet tube (24) and the inner lining (39).

9. The apparatus according to claim 8, wherein the proximal flaps (146) are shaped to define multiple flap openings (161), and wherein the pump-outlet tube (24) and the inner lining (39) are heat welded to one another at least partly via the flap openings (161).

10. The apparatus according to claim 8, wherein a glass-transition temperature of the inlet guard (145) is higher than the respective glass-transition temperatures of each of the inner lining (39) and the pump-outlet tube (24).

11. The apparatus according to claim 10, wherein:
the inner lining (39) is made of a polyurethane,
the pump-outlet tube (24) is made of a polyether block amide, and
the inlet guard (145) is made of a polyether ether ketone;
or
the inner lining (39) and the pump-outlet tube (24) are made of the same type or different types of polyurethane, and
the inlet guard (145) is made of a polyether ether ketone.

12. The apparatus according to any one of claims 1-5, further comprising:
an axial shaft (92) configured to rotate;
a radial bearing (118) configured to radially stabilize the axial shaft (92) while the axial shaft (92) rotates; and
a bearing housing (118h), which houses the radial bearing (118) and is coupled to the frame (34) distally to the frame (34),
wherein the impeller (50) is disposed over the axial shaft (92), and
wherein the inlet guard (145) is coupled to the bearing housing (118H); preferably
wherein the inlet guard (145) comprises multiple distal flaps (148), which are coupled to the bearing housing (118H).

13. The apparatus according to any one of claims 1-5,
wherein the distal portion (40) of the frame (34) is frustoconical, and
wherein the main body of the inlet guard (145) is frustoconical and is fixed over the distal portion (40) of the frame (34); preferably
wherein the inlet guard (145) comprises a rolled sheet (144) of material.

14. The apparatus according to any one of claims 1-5, wherein the main body of the inlet guard (145) is flat and is disposed within the frame (34).

15. The apparatus according to claim 14, further comprising an axial shaft (92) configured to rotate,
wherein the impeller (50) is disposed over the axial shaft (92), and
wherein the main body of the inlet guard (145) is perpendicular to the axial shaft (92).
